# EUROPEAN PATENT APPLICATION

(11) **EP 2 551 257 A1**
(43) Date of publication of application: **30.01.2013**
(21) Application number: 11384003.7
(22) Date of filing: 28.07.2011
(51) Int. Cl.: C07C 57/145, C07C 59/265, C07C 65/05, C07C 233/18, C07C 275/02

(54) **Co-crystals of agomelatine with co-crystal-formers**

(71) Applicant: Laboratorios del. Dr. Esteve, S.A., 08041 Barcelona (ES)
(72) Inventor: Plata Salaman, Carlos Ramon, 08950 Esplugues de Llobregat (Barcelona) (ES); Tesson, Nicolas, 08902 L'Hospitalet de Llobregat (Barcelona) (ES); Cárdenas Romaña, Lydia, 08042 Barcelona (ES); Casas Cartagena, Marçal, 08911 Badalona (Barcelona) (ES)
(74) Representative: Peters, Hajo

(57) **Abstract**

The present invention relates to co-crystals of agomelatine and co-crystal-formers, processes for preparation of the same and their use as medicament or in pharmaceutical formulations, more particularly for the treatment of depression.

## Description

The present invention relates to co-crystals of agomelatine and co-crystal-formers, processes for preparation of the same and their use as medicament or in pharmaceutical formulations, more particularly for the treatment of depression.

Depression, the impairment of affectivity including depressive episodes is a major threat to society. It involves state of low mood and aversion to activity and periods of low self esteem. It is a disorder which might strongly and negatively affect the life of the person suffering from it as well as those near to him hitting all major areas of personal life with suicide being a looming risk. Treatment of depression therefore is a challenge of high interest to society.

The overall aim of the current invention was thus to provide a convenient way of treating depression or to improve an existing one.

Agomelatine is a dual melatoninergic agonist and serotonergic antagonist indicated for the treatment of major depression and has been reported to have a reduced level of sexual side effects as well as discontinuation effects compared to some other antidepressants. Agomelatine may also have positive effects on sleep. Agomelatine, N-[2-(7-methoxynaphthalen-1-yl)ethyl]acetamide has the following formula:

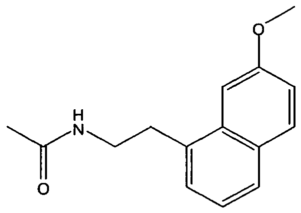

Agomelatine is a melatonergic agonist (MT1 and MT2 receptors) and 5-HT_{2C} antagonist. The 5-HT2c receptor is considered a relevant target with regard to antidepressant therapy. Binding studies indicate that it has no effect on monoamine uptake and no affinity for α, β adrenergic, histaminergic, cholinergic, dopaminergic and benzodiazepine receptors.

Crystals of agomelatine are disclosed in Cryst. Growth Des., 2011, 11 (2), pp. 466-471.

Agomelatine is practically insoluble in purified water (<0,1 mg/ml) but freely soluble (>100 mg/ml) in various organic solvents (96% ethanol, methanol, methylene chloride).

One possible solution of the overall problem of providing new ways of treating depression is the provision of new co-crystals of agomelatine.

For example, a more water soluble co-crystal could be more convenient for use in a pharmaceutical formulation for faster or immediate release in an aqueous environment. Furthermore, it would be advantageous if the co-crystal would be sufficiently stable, both in the solid state and in the solution, and it would also be a benefit if it would be obtainable in solid crystalline form.

Accordingly, it would be desirable to find a solid, particularly crystalline from, a co-crystal of agomelatine and a co-crystal-former that is more soluble in water and/or sufficiently stable against formation of impurities and/or conveniently produced.

In order to increase absorption rate/extent and hence bioavailability of agomelatine, one of the objects of the present invention is thus improving the aqueous solubility and dissolution rate of agomelatine by providing new drugable forms of agomelatine. Another - maybe combined - advantage would be retardation of the precipitation of dissolved agomelatine.

A parallel goal would also be the provision of new means of improving the properties of agomelatine, especially in regard to the treatment of depression, by providing new drugable forms of agomelatine. Especially desirable improvements/advantages of the new drugable form would include any single one of the following or even more preferable even more than one of the following advantages below:
- improvement of physicochemical properties in order to facilitate the formulation, the manufacture, or to enhance the absorption and/or the bioavailability, and/or
- providing new forms of agomelatine with co-formers having a beneficial pharmacological effect in itself, thus allowing for a highly efficient dose/weight relation of the final active principle, and/or
- being easily obtainable, easy to manufacture, and/or
- being obtainable in a solid/stable crystalline form, and/or
- allowing more flexibility in formulating, or facilitating its formulation, and/or
- being highly soluble, thus allowing better dissolution rates, especially if dissolving in an aqueous physiological surrounding, and/or
- improve stability of the co-crystal - also in a pharmaceutical formulation - in comparison with agomelatine alone, and/or
- allowing new routes of administration, and/or
- allowing retardation of the precipitation of dissolved agomelatine, and/or
- reducing adverse drug reactions, and/or
- allowing the use of a lower therapeutic dose of agomelatine, and/or
- showing improvements in the treatment of a patient suffering from depression, and/or
- providing a rapid onset of the effect, and/or
- increasing treatment adherence or compliance of a patient suffering from depression.
   Any and all of these advantages would be desirable. Therefore it would be advantageous if more than one (most preferably many) of these advantages would be fulfilled by the new drugable form.

The objective in general was achieved by providing a new co-crystal comprising agomelatine either as a free base or as a physiologically salt and co-crystal-former either as a free base or as a physiologically salt.

This was quite an achievement as it is well-known that attempts to obtain co-crystals of certain molecules often encounter a huge number of chemical difficulties to be overcome.

These new co-crystals show improved properties if compared to agomelatine alone. Co-crystals thus obtained have a specific stoichiometry which depends upon the structure of each co-crystal-former. Under the proper circumstance this is also another advantage of this new solid drugable form, possibly achieving some modulation of the pharmacological effects.

Specifically, in many cases the physico-chemical properties are improved. Formulating these co-crystals seems to turn-out even easier with such a respective solid to manipulate and with many of the co-crystals according to the invention showing an enhanced stability. The solubility may also be enhanced. This was shown in an improved intrinsic dissolution.

Intrinsic dissolution is the dissolution rate of a drug substance under the condition of constant surface area and is used to compare dissolution properties of different drug substances or different solid forms of the same drug substance. Solid forms with different intrinsic dissolution rates can be used to make drug products with different properties. For example, a solid form with a rapid intrinsic dissolution rate could be used to make an immediate release formulation. By comparison, a solid form with a slow dissolution rate could be used to make a sustained release drug formulation. Thus, the dissolution rate of a co-crystal when compared to that of the API alone can be used as an indication of whether a drug formulation with a faster or slower release profile could be made, and therefore, can be used to prepare a beneficial pharmaceutical product (see Examples 4, 5 and 6).

Another advantage seeming to have been achieved is that the combination of one active principle and a co-former (urea, maleic, gentisic, citric,....) into one unique species seems to allow for a better Pharmacokinetic/Pharmacodynamic (PKPD) which helps in the treatment of depression (e.g. by likely allowing lowering of the dose, or increasing the compliance, or reducing side effects).

Thus, the present invention is drawn to a co-crystal (or co-crystals) comprising agomelatine either as a free base or as a physiologically salt and at least one co-crystal-former either as a free base or as a physiologically acceptable salt.

The following proviso may apply:
- a co-crystal (or co-crystals) of agomelatine with acetic acid is (are) excluded.

The following proviso may apply:
- a co-crystal (or co-crystals) of agomelatine with ethylene glycol is (are) excluded.

The following combined proviso may thus also apply:
- a co-crystal (or co-crystals) of agomelatine with acetic acid, and
- a co-crystal (or co-crystals) of agomelatine with ethylene glycol is/are excluded.

"Co-crystal" as used herein is defined as a crystalline material comprising two or more compounds at ambient temperature (20 to 25°C, preferably 20°C), of which at least two are held together by weak interaction, wherein at least one of the compounds is a co-crystal former. Weak interaction is being defined as an interaction which is neither ionic nor covalent and includes for example: hydrogen bonds, van der Waals forces, and π-π-interactions. The co-crystals may include one or more solvate molecules in the crystalline lattice. Just for the sake of clarity the distinction between crystalline salt and a co-crystal has to be stressed here. An API bound to another compound forming a salt by means of ionic interaction can be considered as one "compound" according to the invention, but it cannot be considered as two compounds by itself.

In scientific literature there currently is some discussion on the proper use of the word co-crystal (see for example Desiraju, Cryst. Eng. Comm., 2003, 5(82), 466-467 and Dunitz, CrystEngComm, 2003, 5(91), 506). A recent article by Zaworotko (Zaworotko, Crystal Growth & Design, Vol. 7, No. 1, 2007, 4-9) gives a definition of co-crystal which is in line with the definition given above.

"Co-crystal-former" as used herein is defined as a component with which agomelatine is able to form co-crystals. The co-crystal-former is part of the crystal lattice. Typically, co-crystal-formers will have the ability to form complementary non-covalent interactions with the active agent, for example the ability to form hydrogen bonds with the active agent in the case of co-crystals.

"Depression" is defined as impairment of affectivity including (major) depressive episodes. It is also a state of low mood and aversion to activity.

In one embodiment of the co-crystal according to the invention the co-crystal-former is selected from either carbamides or carbonic acids, preferably is selected from either carbamides or carbonic acids with less than 9 carbon atoms, most preferably is selected from the group consisting of urea, maleic acid, gentisic acid, citric acid or their stereoisomers.

In one further embodiment of the co-crystal according to the invention the co-crystal is selected from agomelatine - urea (1:1), agomelatine - maleic acid (1:1) form A, agomelatine - maleic acid (1:1) form B, agomelatine - gentisic acid (1:1); or agomelatine - citric acid (1:1).

In a further embodiment of the co-crystal according to the invention the molecular ratio between agomelatine and one co-crystal former (or the co-crystal-former itself) is chosen in such a way that if compared to either agomelatine alone and/or to a mixture of agomelatine and the co-crystal former
- the solubility of the co-crystal is increased; and/or
- the dose response of the co-crystal is increased; and/or
- the efficacy of the co-crystal is increased; and/or
- the dissolution of the co-crystal is increased; and/or
- the bioavailability of the co-crystal is increased; and/or
- the stability of the co-crystal is increased; and/or
- the hygroscopicity of the co-crystal is decreased; and/or
- the form diversity of the co-crystal is decreased; and/or
- the morphology of the co-crystal is modulated.

This applies for example also to the choice of ratio between agomelatine (as a free base or as a physiologically salt) and the co-crystal-former (or of the co-crystal-former itself) being chosen in such a way that it fulfils one or more of the above advantages if compared to agomelatine alone or to a comparable mixture of agomelatine and the co-crystal-former.

"Mixture of agomelatine and the co-crystal-former" is defined as a mixture of agomelatine with the co-crystal-former which is only a physical mixture without any coupling forces between the compounds and thus neither includes salts nor another co-crystal.

The term "salt" is to be understood as meaning any form of agomelatine in which it assumes an ionic form or is charged and is coupled with a counter-ion (a cation) or is in solution. By this are also to be understood complexes of agomelatine (or of the co-crystal-former) with other molecules and ions, in particular complexes which are complexed via ionic interactions. This also includes physiologically acceptable salt.

The term "solvate" according to this invention is to be understood as meaning any form of agomelatine (or of the co-crystal-former) in which the compound has attached to it via non-covalent binding a solvent (most likely a polar solvent) especially including hydrates and alcoholates, e.g. mono-hydrate.

In one preferred embodiment of the co-crystal according to the invention the molecular ratio between agomelatine and co-crystal-former is 1:1.

In another preferred embodiment of the co-crystal according to the invention being a co-crystal of agomelatine - urea (1:1) the endothermic sharp peak corresponding to the melting point has an onset at 135 °C, preferably at 134.6 °C, most preferably at 134.64 °C; or

the co-crystal according to the invention being a co-crystal of agomelatine - urea (1:1) shows an X-Ray powder diffraction pattern with peaks [2θ] at 15.59 and 22.81, preferably with peaks [2θ] at 15.59, 19.53, 22.81, 28.04, and 31.43, more preferably with peaks [2θ] at 9.54, 13.34, 15.59, 17.65, 19.53, 21.35, 22.81, 28.04, and 31.43, most preferably with peaks [2θ] at 9.54, 10.70, 11.32, 13.34, 15.59, 17.65, 18.93, 19.13, 19.53, 21.35, 22.04, 22.81, 24.32, 24.81, 25.30, 26.33, 28.04, 29.27, 31.43 and 33.23; or
the co-crystal according to the invention being a co-crystal of agomelatine - urea (1:1) has a monoclinic unit cell with the following dimensions:
a = 9.72 Å,
b = 9.24 Å, and
c = 18.53 Å,
with an angle β of 102.00 °.

In another preferred embodiment of the co-crystal according to the invention being a co-crystal of agomelatine - maleic acid (1:1) form A the endothermic sharp peak corresponding to the melting point has an onset at 68 °C, preferably at 67.8 °C, most preferably at 67.81 °C; or
the co-crystal according to the invention being a co-crystal of agomelatine - maleic acid (1:1) form A shows an X-Ray powder diffraction pattern with peaks [2θ] at 15.93, 25.83, and 26.20, preferably with peaks [2θ] at 14.61, 15.93, 17.63, 23.43, 25.83, and 26.20, more preferably with peaks [2θ] at 8.78, 11.26, 14.61, 15.93, 17.63, 19.69, 23.43, 25.83, and 26.20, most preferably with peaks [2θ] at 8.78, 11.26, 11.70, 12.45, 14.61, 15.44, 15.93, 17.11, 17.63, 19.69, 20.23, 21.18, 22.23, 23.43, 24.40, 25.08, 25.83, 26.20, 27.03, 29.07, 29.50, 30.56, 32.07, 33.31 and 35.68.

In another preferred embodiment of the co-crystal according to the invention being a co-crystal of agomelatine - maleic acid (1:1) form B the endothermic sharp peak corresponding to the melting point has an onset at 73 °C, preferably at 73.2 °C, most preferably at 73.18 °C; or
the co-crystal according to the invention being a co-crystal of agomelatine - maleic acid (1:1) form B shows an X-Ray powder diffraction pattern with peaks [2θ] at 11.31, 15.42, 17.13, 17.30, 18.02, and 24.32, preferably with peaks [2θ] at 8.57, 11.31, 15.42, 17.13, 17.30, 18.02, 18.73, 23.93, 24.32, 25.48, and 26.22, more preferably with peaks [2θ] at 8.57, 11.31, 15.42, 17.13, 17.30, 17.57, 18.02, 18.73, 21.80, 22.93, 23.93, 24.32, 25.48, 26.22, 27.24, and 30.25, most preferably with peaks [2θ] at 7.37, 8.57, 11.31, 13.64, 14.67, 15.42, 15.62, 17.13, 17.30, 17.57, 18.02, 18.73, 19.48, 21.80, 22.24, 22.93, 23.93, 24.32, 25.48, 26.22, 26.53, 26.97, 27.24, 27.57, 28.04, 29.31, 30.25, 31.51, 32.11, 34.27, 35.20, 36.51, 37.31 and 37.98; or
the co-crystal according to the invention being a co-crystal of agomelatine - maleic acid (1:1) form B has a monoclinic unit cell with the following dimensions:
a = 7.51 Å,
b = 20.59 Å, and
c = 12.09 Å,
with an angle β of 99.22 °.

In another preferred embodiment of the co-crystal according to the invention being a co-crystal of agomelatine - gentisic acid (1:1) the endothermic sharp peak corresponding to the melting point has an onset at 108 °C, preferably at 107.9 °C, most preferably at 107.88 °C; or
the co-crystal according to the invention being a co-crystal of agomelatine - gentisic acid (1:1) shows a X-Ray powder diffraction pattern with peaks [2θ] at 18.05, 21.63, and 22.98, preferably with peaks [2θ] at 17.15, 18.05, 21.63, 22.13, 22.98, and 24.46, more preferably with peaks [2θ] at 8.06, 11.66, 16.03, 17.15, 17.60, 18.05, 21.63, 22.13, 22.98, 24.46, and 27.17, most preferably with peaks [2θ] at 8.06, 11.66, 12.60, 14.24, 15.16, 16.03, 16.94, 17.15, 17.60, 18.05, 18.81, 19.39, 20.04, 21.63, 22.13, 22.46, 22.98, 23.33, 24.46, 24.77, 25.64, 26.56, 27.17, 29.27, 30.64, 30.96, 31.61, 32.71 and 36.55; or
the co-crystal according to the invention being a co-crystal of agomelatine - gentisic acid (1:1) has a monoclinic unit cell with the following dimensions:
a = 11.35 Å,
b = 11.04 Å, and
c = 16.80 Å,
with an angle β of 105.00 °.

In another preferred embodiment of the co-crystal according to the invention being a co-crystal of agomelatine - citric acid (1:1) the endothermic sharp peak corresponding to the melting point has an onset at 127 °C, preferably at 127.2 °C, most preferably at 127.16 °C; or
the co-crystal according to the invention being a co-crystal of agomelatine - citric acid (1:1) shows a X-Ray powder diffraction pattern with peaks [2θ] at 5.22, 17.06, 19.36, 22.80, and 27.28, preferably with peaks [2θ] at 5.22, 17.06, 19.36, 20.72, 20.98, 21.87, 22.80, and 27.28, more preferably with peaks [2θ] at 5.22, 10.42, 12.24, 13.74, 15.38, 15.55, 17.06, 17.74, 19.36, 19.77, 20.72, 20.98, 21.87, 22.80, 24.05, 26.57, 27.28, 29.66, and 31.42, most preferably with peaks [2θ] at 5.22, 10.42, 11.58, 12.24, 12.43, 13.74, 14.35, 15.38, 15.55, 17.06, 17.74, 19.36, 19.77, 20.72, 20.98, 21.87, 22.80, 23.15, 24.05, 24.75, 24.99, 25.23, 25.54, 26.26, 26.57, 27.28, 27.88, 28.23, 28.66, 29.66, 31.42, 32.72, 33.25, 33.97, 34.54, 35.01, 36.28, 37.33, 37.98 and 38.44; or
the co-crystal according to the invention being a co-crystal of agomelatine - citric acid (1:1) has a monoclinic unit cell with the following dimensions:
a = 7.84 Å,
b = 33.89 Å, and
c = 8.29 Å,
with an angle β of 109.38 °.

Another aspect of the present invention relates to a process for the production of a co-crystal according to the invention as described above comprising the steps of:
(a) dissolving or suspending agomelatine and co-crystal former in a solvent or mixture of solvents,
(b) optionally mixing or stirring the solution or suspension,
(c) optionally before, during or after step (b) cooling or keeping the mixed solution/suspension to or at ambient temperature or below;
(d) optionally filtering-off and/or washing the resulting solid with a solvent, and
(e) drying the solid optionally at ambient temperature and/or vacuum.

"Ambient temperature" (also known as "room temperature") is defined here as a temperature between 20 and 25°C, preferably being 20°C.

In a preferred embodiment the organic solvent used in step a) in this process is selected from isopropanol, ethanol, toluene, ethyl acetate, acetone, heptane, or a mixture of acetone and heptane.

In another preferred embodiment the organic solvent used in step d) in this process is selected from isopropanol, ethanol, toluene, ethyl acetate, tert-butyl methyl ether, diethyl ether, acetone, heptane, or a mixture of acetone and heptane.

The active principle agomelatine that forms part of the co-crystal according to the invention is a well-known drug and preferably and in the examples the co-crystal-former is physiologically acceptable. Due to this, a further object of the present invention is a medicament comprising at least one co-crystal according to the invention and optionally one or more pharmaceutically acceptable excipients. Thus the invention also relates to a medicament comprising at least one co-crystal according to the invention as described above.

The invention also relates to a pharmaceutical composition comprising a therapeutically effective amount of the co-crystal according to the invention as described above in a physiologically acceptable medium. The invention also relates to a pharmaceutical composition comprising the co-crystal according to the invention and optionally one or more pharmaceutically acceptable excipients.

The invention also relates to a pharmaceutical composition comprising a therapeutically effective amount of the co-crystal according to the invention and optionally one or more pharmaceutically acceptable excipients.

The invention also concerns a medicament or pharmaceutical composition comprising (preferably a therapeutically effective amount of) at least one co-crystal according to the invention as described above and optionally one or more pharmaceutically acceptable excipients for use in the treatment of depression especially in the treatment of major depressive episodes.

In general, in most embodiments in which the co-crystals according to the invention are used (e.g. for the treatment of depression) these co-crystals would be formulated into a convenient pharmaceutical formulation or a medicament. Accordingly a desirable advantage of a co-crystal according to the invention would be showing improved pharmaceutical properties and features for use in such a formulation, especially when compared to agomelatine either as a free base or as a physiologically acceptable salt alone. Thus, the co-crystal according to the invention should desirably show at least one, preferably more, of the following features - especially if used or for use in a convenient pharmaceutical formulation or a medicament:
- to have a very small particle size, e.g. from 300 µm or lower; or
- to be and/or remain essentially free of agglomerates; or
- to be less or not very hygroscopic; or
- to help in formulating controlled release or immediate release formulations; or
- to have a high chemical stability; or
   if given to a patient
- to decrease the inter- and intra-subject variability in blood levels; or
- to show a good absorption rate (e.g. increases in plasma levels or AUC); or
- to show a high maximum plasma concentration (e.g. Cₘₐₓ); or
- to show decreased time to peak drug concentrations in plasma (tₘₐₓ); or
- to show changes in half life of the compound (t_{1/2}), in whichever direction this change is preferably directed.

The medicament or pharmaceutical compositions according to the present invention may be in any form suitable for the application to humans and/or animals, preferably humans including infants, children and adults and can be produced by standard procedures known to those skilled in the art. The medicament of the present invention may for example be administered parenterally, including intramuscular, intraperitoneal, or intravenous injection, transmucosal or sublingual application; or orally, including administration as tablets, pellets, granules, capsules, lozenges, aqueous or oily solutions, suspensions, emulsions, sprays or as reconstituted dry powdered form with a liquid medium.

Typically, the medicaments according to the present invention may contain 1-60 % by weight of one or more of the co-crystals as defined herein and 40-99 % by weight of one or more pharmaceutically acceptable excipients.

The compositions of the present invention may also be administered topically or via a suppository.

The daily dosage for humans and animals (especially adults) may vary depending on factors that have their basis in the respective species or other factors, such as age, sex, weight or degree of illness and so forth. The daily dosage for humans preferably is in the range of 5 to 500 milligrams of agomelatine (or the equivalent in the co-crystal) or preferably 10 to 250 milligrams of agomelatine (or the equivalent in the co-crystal) or more preferably 25 to 50 milligrams of agomelatine (or the equivalent in the co-crystal). This can be administered during one or several intakes per day, preferably once or twice daily e.g. in a dosage of 25 mg of agomelatine (or the equivalent in the co-crystal).

The daily dosage for humans preferably is in the range of 5 to 500 milligrams of co-crystal according to the invention or of the respective part of the co-crystal according to the invention, preferably in the range of 10 to 250 milligrams, most preferably in the range of 25 to 50 milligrams preferably to be administered during one or several (preferably one or two) intakes per day.

A further aspect of the invention relates to the use of a co-crystal according to the invention as described above for the treatment of depression, especially for the treatment of major depressive episodes. Preferably this use is provided in the form of a medicament or a pharmaceutical composition according to the invention as described above.

Another aspect of the invention relates to the use of a co-crystal according to the invention as described above in the manufacture of a medicament for the treatment of depression; especially for the treatment of major depressive episodes.

The invention also relates to a co-crystal according to the invention as described above for use in the treatment of depression, especially in the treatment of major depressive episodes.

Another object of the current invention is a method of treatment of depression or of major depressive episodes by providing to a patient in need thereof a sufficient amount of a co-crystal according to the invention as described above. Preferably the co-crystal according to the invention is provided in physiologically suitable form like e.g. in the form of a medicament or a pharmaceutical composition according to the invention as described above e.g. comprising 25 to 50 milligrams of agomelatine (or the equivalent in the co-crystal).

The present invention is illustrated below with the help of the following figures and examples. These illustrations are given solely by way of example and do not limit the invention.

### Brief description of the figures:

**Figure 1****:** Differential scanning calorimetry (DSC) analysis of agomelatine - urea (1:1) co-crystal
**Figure 2****:** Thermogravimetric analysis (TGA) of agomelatine - urea (1:1) co-crystal
**Figure 3****:** X-ray powder diffraction (XRPD) pattern of agomelatine - urea (1:1) co-crystal
**Figure 4****:** Crystal structure of agomelatine - urea (1:1) co-crystal
**Figure 5****:** Differential scanning calorimetry (DSC) analysis of agomelatine - maleic acid (1:1) co-crystal, form A
**Figure 6****:** Thermogravimetric analysis (TGA) of agomelatine - maleic acid (1:1) co-crystal, form A
**Figure 7****:** X-ray powder diffraction (XRPD) pattern of agomelatine - maleic acid (1:1) co-crystal, form A
**Figure 8****:** Differential scanning calorimetry (DSC) of agomelatine - maleic acid (1:1) co-crystal, form B
**Figure 9****:** Thermogravimetric analysis (TGA) of agomelatine - maleic acid (1:1) co-crystal, form B
**Figure 10****:** X-ray powder diffraction (XRPD) pattern of agomelatine - maleic acid (1:1) co-crystal, form B
**Figure 11****:** Crystal structure of agomelatine - maleic acid (1:1) co-crystal, form B
**Figure 12****:** Differential scanning calorimetry (DSC) analysis of agomelatine - gentisic acid (1:1) co-crystal
**Figure 13****:** Thermogravimetric analysis (TGA) of agomelatine - gentisic acid (1:1) co-crystal
**Figure 14****:** X-ray powder diffraction (XRPD) pattern of agomelatine - gentisic acid (1:1) co-crystal
**Figure 15****:** Crystal structure of agomelatine - gentisic acid (1:1) co-crystal
**Figure 16****:** Differential scanning calorimetry (DSC) analysis of agomelatine - citric acid (1:1) co-crystal
**Figure 17****:** Thermogravimetric analysis (TGA) of agomelatine - citric acid (1:1) co-crystal
**Figure 18****:** X-ray powder diffraction (XRPD) pattern of agomelatine - citric acid (1:1) co-crystal
**Figure 19****:** Crystal structure of agomelatine - citric acid (1:1) co-crystal
**Figure 20****:** Intrinsic Dissolution Rate (IDR) test of the co-crystals from examples 4 and 5 at HCl 0.1
**Figure 21****:** Intrinsic Dissolution Rate (IDR) test of the co-crystals from examples 4 and 5 at buffer pH 6.8

### EXAMPLES

### Example 1: Agomelatine - Urea (1:1) co-crystal

### Slurrying in isopropanol

To a round-bottom flask equipped with magnetic stirrer containing agomelatine (489 mg, 2.01 mmol) and urea (121 mg, 2.01 mmol, 1.0 eq), was added isopropanol (6 mL). The resultant suspension was stirred at room temperature for 20 hours. The solid was filtered with a sintered funnel (porosity 4), washed with cold isopropanol (0.5 mL) and dried under vacuum at room temperature to give co-crystal of agomelatine - urea (1:1) as a white solid (252 mg, 41 % yield).

### ¹H NMR

Proton nuclear magnetic resonance analyses were recorded in deuterated dimethyl sulfoxide (DMSO) in a Varian Mercury 400 spectrometer, equipped with a broadband probe ATB 1H/19F/X of 5 mm. Spectra were acquired dissolving 5-10 mg of sample in 0.6 mL of deuterated solvent.

¹H NMR (dmso, 400 MHz): δ = 8.09 (m, 1H); 7.82 (d, *J* = 9.0 Hz, 1H); 7.71 (d, *J* = 8.0 Hz, 1H); 7.60 (d, *J* = 2.7 Hz, 1H); 7.31 (dd, *J* = 7.0 Hz and 1.2 Hz, 1H); 7.26 (dd, *J* = 7.0 Hz and 8.0 Hz, 1H); 7.16 (dd, *J* = 9.0 Hz and 2.7 Hz, 1H); 5.38 (s br, 4H); 3.94 (s, 3H); 3.27-3.38 (m, 2H); 3.14-3.07 (m, 2H); 1.81 (s, 3H).

### IR

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3484.7 (s), 3367.2 (s), 3276.7 (s), 3183.6 (s), 3096.7 (m), 2944.4 (m), 1680.5 (s), 1650.3 (s), 1626.0 (s), 1600.3 (s), 1569.5 (s), 1510.9 (m), 1449.1 (m), 1305.6 (m), 1251.3 (s), 1216.3 (s), 1181.3 (m), 1026.4 (m), 827.7 (s), 758.1 (m) cm⁻¹.

### DSC

DSC analyses were recorded with a Mettler DSC822^{e}. A sample of 2.8420 mg was weighed into 40 µL aluminium crucible with a pinhole lid and was heated, under nitrogen (50 mL/min), at 10 °C/min from 30 to 200 °C.

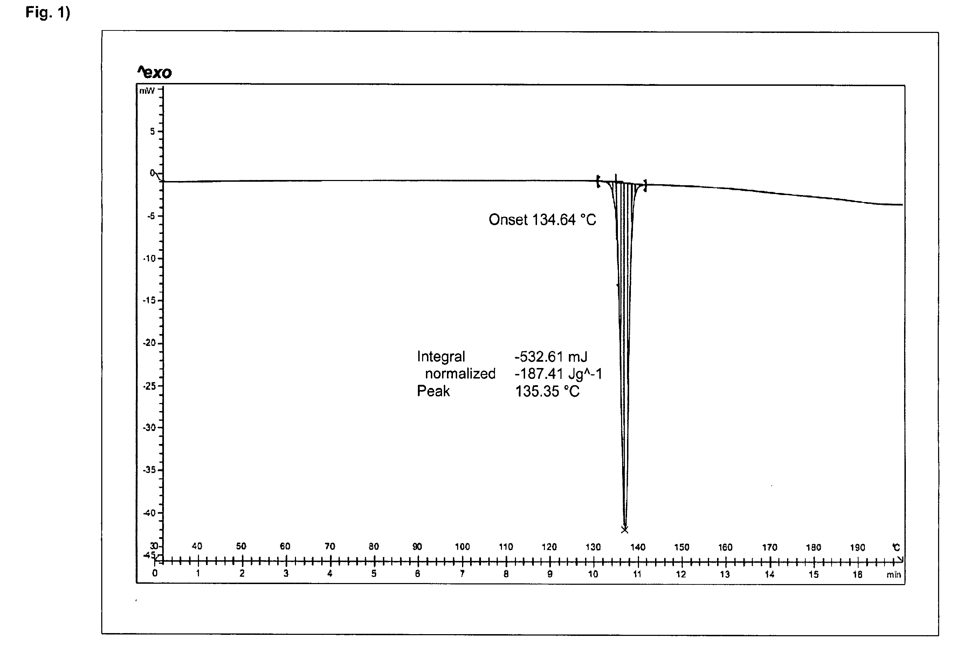

The novel type of crystal of the present invention is characterized in that the endothermic sharp peak corresponding to the melting point has an onset at 134.64 °C (fusion enthalpy - 187.41 J/g), measured by DSC analysis (10 °C/min) (see figure 1).

### TG

Thermogravimetric analyses were recorded in a thermogravimetric analyzer Mettler TGA/SDTA851^{e}. A sample of 2.5106 mg was weighed into a 70 µL alumina crucible with a pinhole lid and was heated at 10 °C/min from 30 to 200 °C, under nitrogen (50 mL/min).

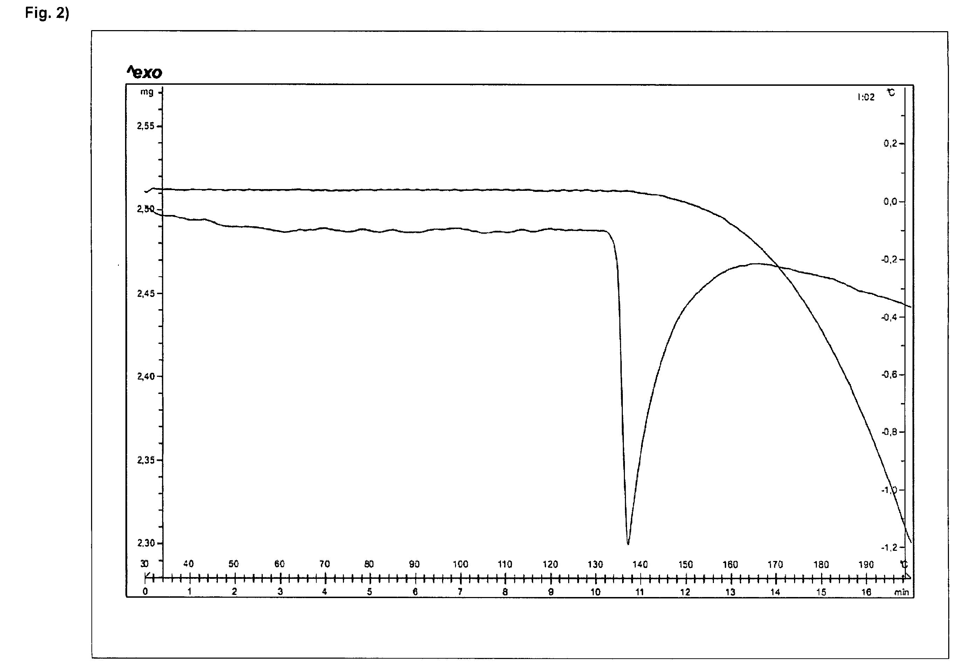

The TG analysis of the co-crystal of agomelatine - urea (1:1) according to the invention shows no weight loss at temperatures lower than the melting point (see figure 2).

### XRPD

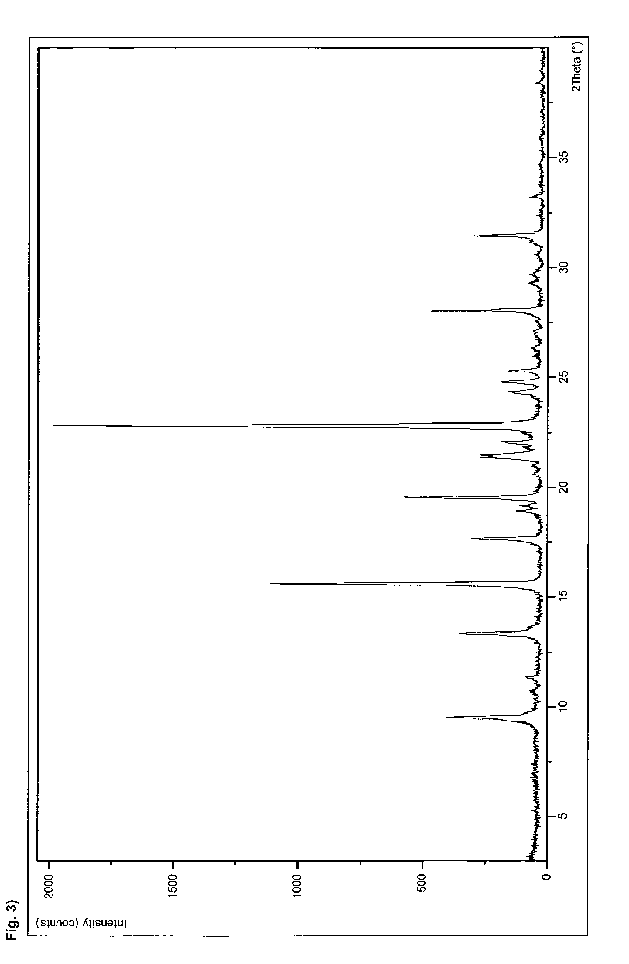

XRPD analysis was performed using a Philips X'Pert diffractometer with Cu K_{α} radiation in Bragg-Brentano geometry. The system is equipped with a monodimensional, real time multiple strip detector. The diffractogram was recorded from 3° to 40° (2θ) at a scan rate of 17.6° per minute (see figure 3).

List of selected peaks (only peaks with relative intensity greater than or equal to 1 % are indicated):

| **2**θ **(°)** | **d (**Å**)** | **l (%)** | | **2**θ **(°)** | **d (**Å**)** | **l (%)** |
|---|---|---|---|---|---|---|
| 9.54 | 9.27 | 17 | | 22.04 | 4.03 | 6 |
| 10.70 | 8.26 | 1 | | 22.81 | 3.90 | 100 |
| 11.32 | 7.81 | 2 | | 24.32 | 3.66 | 5 |
| 13.34 | 6.64 | 17 | | 24.81 | 3.59 | 8 |
| 15.59 | 5.68 | 56 | | 25.30 | 3.52 | 5 |
| 17.65 | 5.02 | 14 | | 26.33 | 3.38 | 1 |
| 18.93 | 4.69 | 5 | | 28.04 | 3.18 | 23 |
| 19.13 | 4.64 | 4 | | 29.27 | 3.05 | 2 |
| 19.53 | 4.54 | 28 | | 31.43 | 2.85 | 20 |
| 21.35 | 4.16 | 10 | | 33.23 | 2.70 | 2 |

### Single crystal X-ray diffraction

The crystal structure of co-crystal of agomelatine - urea (1:1) has been determined from single crystal X-ray diffraction data. The colourless prism used (0.26 × 0.16 × 0.13 mm) was recovered from a slurrying of agomelatine and urea in isopropanol.

Analysis was performed at room temperature using a Bruker Smart Apex diffractometer with graphite monochromated Mo K_{α} radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0).

The structure was solved with direct methods and least-squares refinement of Fₒ² against all measured intensities was carried out (program used: SHELXTL-NT 6.1). All non-hydrogen atoms were refined with anisotropic displacement parameters.

Relevant structural data:

| Crystal system | Monoclinic |
|---|---|
| Space group | *P*2₁/*n* |
| a (Å) | 9.7284(10) |
| b (Å) | 9.2401(9) |
| c (Å) | 18.5733(19) |
| β (°) | 101.997(2) |
| Volume (Å³) | 1633.1(3) |
| Z | 4 |
| D calc. (Mg/m³) | 1.234 |
| N. of refl. | 4001 |
| Refl. with I > 2σ(I) | 2057 |
| R (I > 2σ(I)) | 0.0554 |

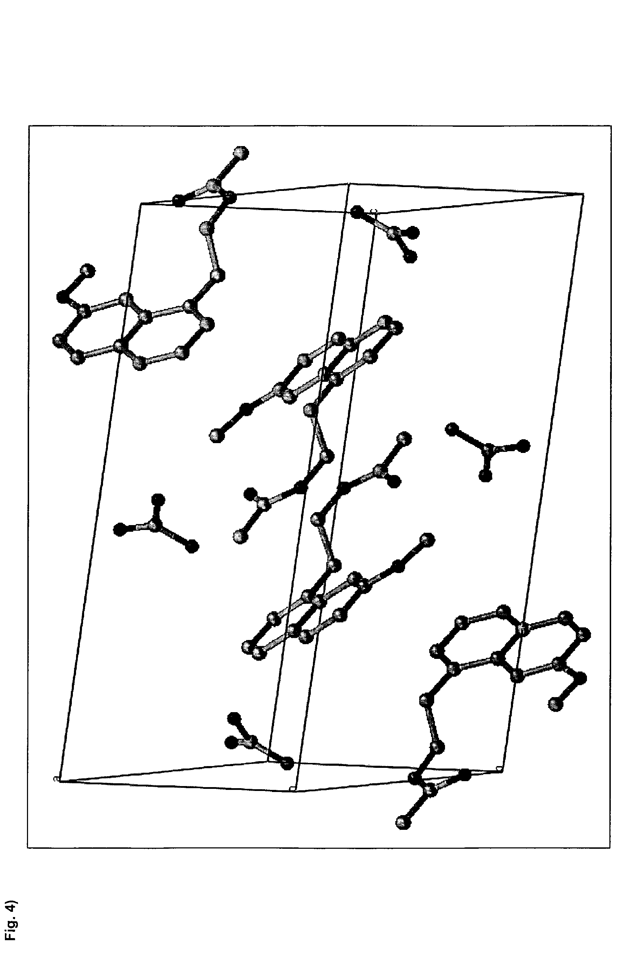

The unit cell contents of this crystal structure are depicted in figure 4 (hydrogen atoms have been omitted for clarity; program used: *Mercury* 2.3, C.F. Macrae, I.J. Bruno, J.A. Chisholm, P.R. Edgington, P. McCabe, E. Pidcock, L. Rodriguez-Monge, R. Taylor, J. van de Streek and P.A. Wood, J. Appl. Cryst., 41, 2008, 466-470).

Positions of peaks in XRPD diffractogram simulated from single crystal data are almost identical to those in the experimental one presented above.

### Example 2: Agomelatine - Maleic acid (1:1) co-crystal, form A

### Wet grinding with ethanol (solvent assisted grinding)

To a 2 mL eppendorf containing agomelatine (50 mg, 0.21 mmol), maleic acid (24 mg, 0.21 mmol) and three 4 mm stainless steel grinding balls, two drops of ethanol were added. The reactor was stirred 45 minutes at a rate of 30 Hz (3 × 15 minutes) giving co-crystal of agomelatine - maleic acid (1:1), form A, as a light yellow solid (74 mg, quantitative yield).

### Precipitation in ethyl acetate

To a vial equipped with magnetic stirrer containing agomelatine (243 mg, 1.00 mmol) and maleic acid (97 mg, 0.83 mmol), was added ethyl acetate (1 mL). The resultant solution was stored in a freezer at a temperature of approximately -20 °C for 5 days. The white precipitate formed was filtered with a sintered funnel (porosity 4) and dried under vacuum at room temperature to give co-crystal of agomelatine - maleic acid (1:1), form A, as a light yellow solid (158 mg, 53 % yield).

### ¹H NMR

Proton nuclear magnetic resonance analyses were recorded in deuterated methanol (MeOD) in a Varian Mercury 400 spectrometer, equipped with a broadband probe ATB 1H/19F/X of 5 mm. Spectra were acquired dissolving 5-10 mg of sample in 0.6 mL of deuterated solvent.

¹H NMR (MeOD, 400 MHz): δ = 7.74 (d, *J* = 9.0 Hz, 1H); 7.65 (d, *J* = 8.0 Hz, 1H); 7.52 (d, *J* = 2.3 Hz, 1H); 7.30 (d, *J* = 6.8 Hz, 1H); 7.23 (dd, *J* = 6.8 Hz and 8.0 Hz, 1H); 7.12 (dd, *J* = 9.0 Hz and 2.3 Hz, 1H); 6.31 (s, 2H); 3.96 (s, 3H); 3.49 (dd, *J* = 6.6 Hz and 8.8 Hz, 2H); 3.22 (dd, *J* = 6.6 Hz and 8.8 Hz, 2H); 1.93 (s, 3H).

### IR

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3282.3 (s), 3129.1 (m), 3007.9 (m), 2944.3 (m), 2889.2 (m), 2838.8 (m), 2431.7 (s,br), 1903.9 (m), 1874.5 (m), 1717.4 (s), 1635.3 (m,br), 1431.6 (m), 1375.3 (m), 1336.3 (m), 639.6 (s), 596.0 (s), 475.4 (s) cm⁻¹.

### DSC

DSC analyses were recorded with a Mettler DSC822^{e}. A sample of 9.5820 mg was weighed into 40 µL aluminium crucible with a pinhole lid and was heated, under nitrogen (50 mL/min), at 10 °C/min from 30 to 300 °C.

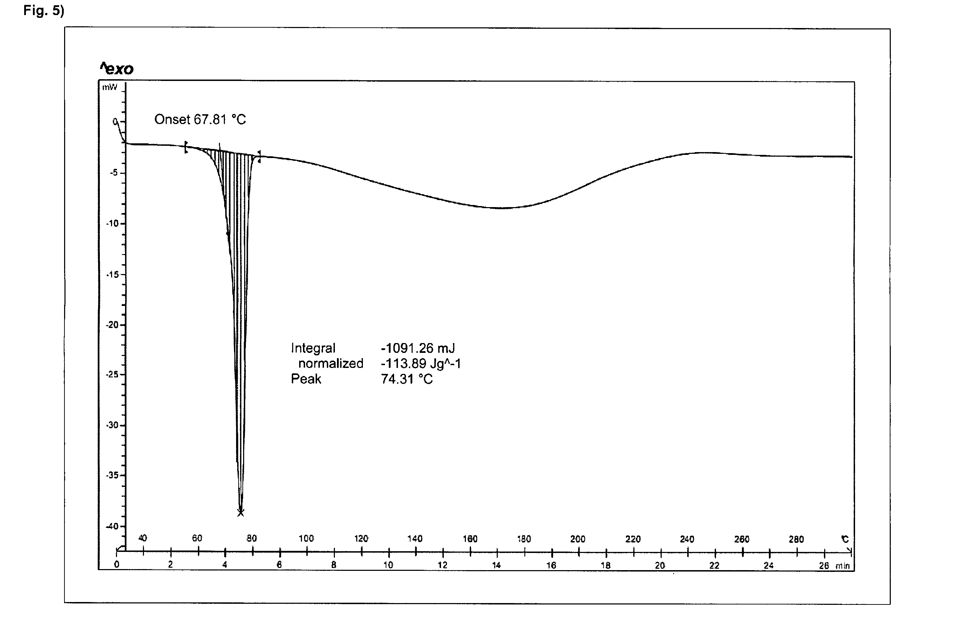

The novel type of crystal of the present invention is characterized in that the endothermic sharp peak corresponding to the melting point has an onset at 67.81 °C (fusion enthalpy - 113.89 J/g), measured by DSC analysis (10 °C/min) (see figure 5).

### TG

Thermogravimetric analyses were recorded in a thermogravimetric analyzer Mettler TGA/SDTA851^{e}. A sample of 18.4406 mg was weighed into a 70 µL alumina crucible with a pinhole lid and was heated at 10 °C/min from 30 to 300 °C, under nitrogen (50 mL/min).

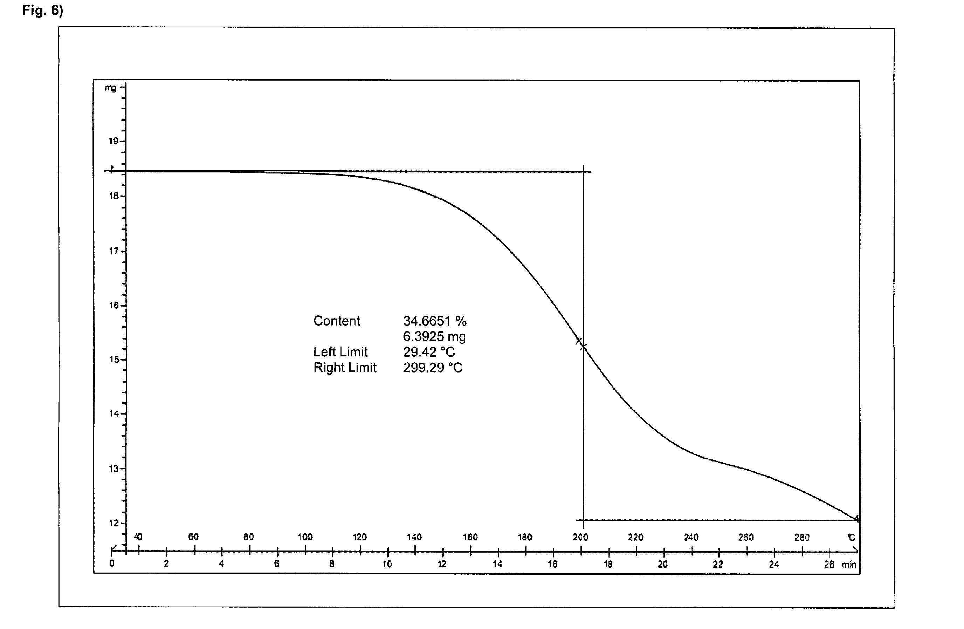

The TG analysis of the co-crystal of agomelatine - maleic acid (1:1), form A, according to the invention shows no weight loss at temperatures lower than the melting point (see figure 6).

### XRPD

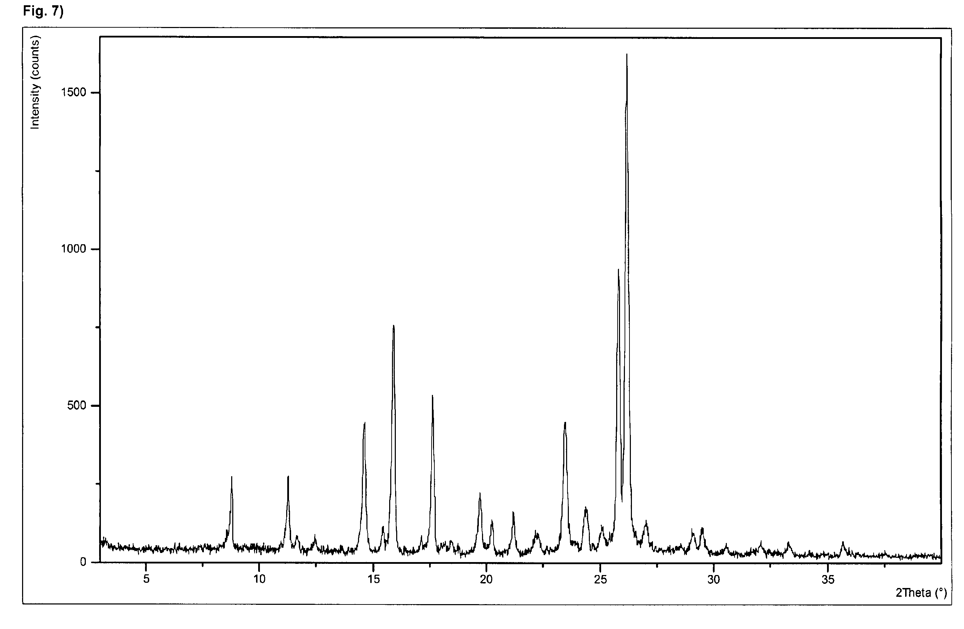

XRPD analysis was performed using a Philips X'Pert diffractometer with Cu K_{α} radiation in Bragg-Brentano geometry. The system is equipped with a monodimensional, real time multiple strip detector. The diffractogram was recorded from 3° to 40° (2θ) at a scan rate of 17.6° per minute (see figure 7).

List of selected peaks (only peaks with relative intensity greater than or equal to 1 % are indicated):

| **2**θ **(°)** | **d (**Å**)** | **l (%)** | | **2θ (°)** | **d (**Å**)** | **l (%)** |
|---|---|---|---|---|---|---|
| 8.78 | 10.06 | 14 | | 23.43 | 3.80 | 26 |
| 11.26 | 7.86 | 15 | | 24.40 | 3.65 | 7 |
| 11.70 | 7.56 | 2 | | 25.08 | 3.55 | 3 |
| 12.45 | 7.11 | 2 | | 25.83 | 3.45 | 56 |
| 14.61 | 6.06 | 26 | | 26.20 | 3.40 | 100 |
| 15.44 | 5.74 | 5 | | 27.03 | 3.30 | 5 |
| 15.93 | 5.56 | 44 | | 29.07 | 3.07 | 3 |
| 17.11 | 5.18 | 2 | | 29.50 | 3.03 | 4 |
| 17.63 | 5.03 | 33 | | 30.56 | 2.93 | 2 |
| 19.69 | 4.51 | 12 | | 32.07 | 2.79 | 2 |
| 20.23 | 4.39 | 6 | | 33.31 | 2.69 | 2 |
| 21.18 | 4.19 | 8 | | 35.68 | 2.52 | 2 |
| 22.23 | 4.00 | 3 | | | | |

### Example 3: Agomelatine - Maleic acid (1:1) co-crystal, form B

### Slurrying in toluene

To an assay tube equipped with magnetic stirrer containing agomelatine (282 mg, 1.16 mmol) and maleic acid (133 mg, 1.15 mmol), was added toluene (2 mL). The resultant suspension was stirred at room temperature for 16 hours. The solid was filtered with a sintered funnel (porosity 4), washed with toluene and dried under vacuum at room temperature to give co-crystal of agomelatine - maleic acid (1:1), form B, as a white solid (334 mg, 80 % yield).

### Wet grinding with ethyl acetate (solvent assisted grinding)

To a 2 mL eppendorf containing agomelatine (69 mg, 0.28 mmol), maleic acid (32 mg, 0.28 mmol) and three 4 mm stainless steel grinding balls, two drops of ethyl acetate were added. The reactor was stirred 45 minutes at a rate of 30 Hz (3 × 15 minutes) giving co-crystal of agomelatine - maleic acid (1:1), form B, as a white solid (101 mg, quantitative yield).

The same experiment was carried out with isopropanol and methyl isobutyl ketone.

### ¹H NMR

Proton nuclear magnetic resonance analyses were recorded in deuterated methanol (MeOD) in a Varian Mercury 400 spectrometer, equipped with a broadband probe ATB 1H/19F/X of 5 mm. Spectra were acquired dissolving 5-10 mg of sample in 0.6 mL of deuterated solvent.

¹H NMR (MeOD, 400 MHz): δ = 7.74 (d, *J* = 9.0 Hz, 1H); 7.65 (d, *J* = 8.0 Hz, 1H); 7.53 (d, *J* = 2.3 Hz, 1H); 7.31 (d, *J* = 7.0 Hz, 1H); 7.24 (*J* = 8.0 Hz and 7.0 Hz, 1H); 7.12 (dd, *J* = 9.0 Hz and 2.3 Hz, 1H); 6.31 (s, 2H); 3.96 (s, 3H); 3.49 (dd, *J* = 6.6 Hz and 8.8 Hz, 2H); 3.22 (dd, *J* = 6.6 Hz and 8.8 Hz, 2H); 1.93 (s, 3H).

### IR

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3318.4 (s), 3126.1 (m), 3063.1 (m), 2978.3 (m), 2463.7 (m,br), 1724.9 (s), 1649.0 (s), 1385.1 (m), 1317.8 (m), 1269.6 (m), 1032.6 (m), 944.0 (m), 861.5 (s), 820.1 (s), 751.0 (s), 636.8 (s), 594.3 (s) cm⁻¹.

### DSC

DSC analyses were recorded with a Mettler DSC822^{e}. A sample of 2.4180 mg was weighed into 40 µL aluminium crucible with a pinhole lid and was heated, under nitrogen (50 mL/min), at 10 °C/min from 30 to 300 °C.

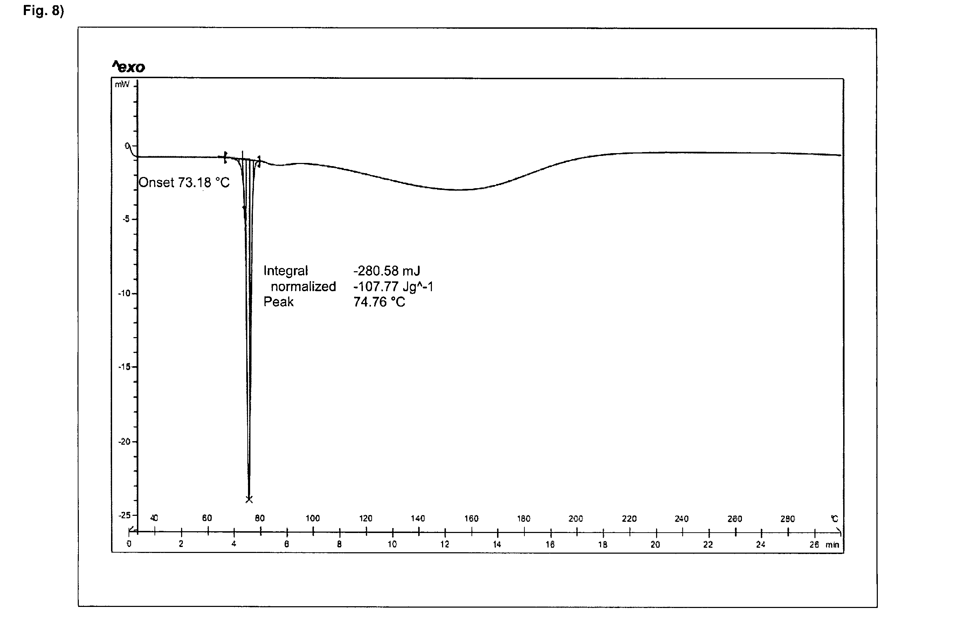

The novel type of crystal of the present invention is characterized in that the endothermic sharp peak corresponding to the melting point has an onset at 73.18 °C (fusion enthalpy - 107.77 J/g), measured by DSC analysis (10 °C/min) (see figure 8).

### TG

Thermogravimetric analyses were recorded in a thermogravimetric analyzer Mettler TGA/SDTA851^{e}. A sample of 7.2406 mg was weighed into a 70 µL alumina crucible with a pinhole lid and was heated at 10 °C/min from 30 to 300 °C, under nitrogen (50 mL/min).

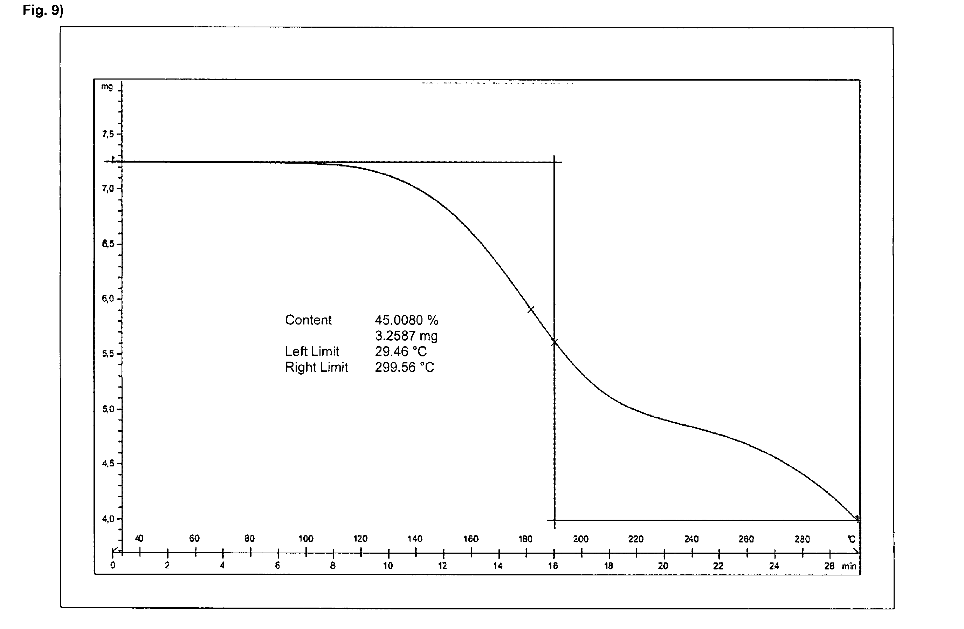

The TG analysis of the co-crystal of agomelatine - maleic acid (1:1), form B, according to the invention shows no weight loss at temperatures lower than the melting point (see figure 9).

### XRPD

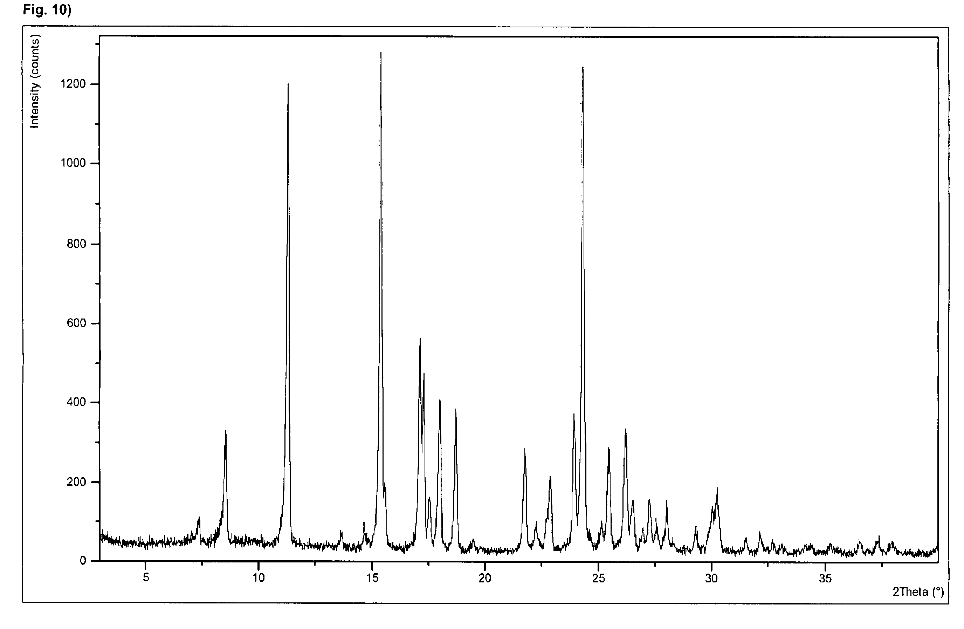

XRPD analysis was performed using a Philips X'Pert diffractometer with Cu K_{α} radiation in Bragg-Brentano geometry. The system is equipped with a monodimensional, real time multiple strip detector. The diffractogram was recorded from 3° to 40° (2θ) at a scan rate of 17.6° per minute (see figure 10).

List of selected peaks (only peaks with relative intensity greater than or equal to 1 % are indicated):

| **2**θ **(°)** | **d (**Å**)** | **l (%)** | | **2θ (°)** | **d (**Å**)** | **l (%)** |
|---|---|---|---|---|---|---|
| 7.37 | 11.99 | 5 | | 24.32 | 3.66 | 97 |
| 8.57 | 10.32 | 22 | | 25.48 | 3.50 | 20 |
| 11.31 | 7.82 | 91 | | 26.22 | 3.40 | 23 |
| 13.64 | 6.49 | 3 | | 26.53 | 3.36 | 8 |
| 14.67 | 6.04 | 3 | | 26.97 | 3.31 | 3 |
| 15.42 | 5.75 | 100 | | 27.24 | 3.27 | 10 |
| 15.62 | 5.67 | 9 | | 27.57 | 3.24 | 4 |
| 17.13 | 5.17 | 40 | | 28.04 | 3.18 | 8 |
| 17.30 | 5.13 | 34 | | 29.31 | 3.05 | 4 |
| 17.57 | 5.05 | 10 | | 30.25 | 2.95 | 12 |
| 18.02 | 4.92 | 30 | | 31.51 | 2.84 | 3 |
| 18.73 | 4.74 | 27 | | 32.11 | 2.79 | 4 |
| 19.48 | 4.56 | 2 | | 34.27 | 2.62 | 1 |
| 21.80 | 4.08 | 18 | | 35.20 | 2.55 | 1 |
| 22.24 | 4.00 | 5 | | 36.51 | 2.46 | 2 |
| 22.93 | 3.88 | 13 | | 37.31 | 2.41 | 2 |
| 23.93 | 3.72 | 28 | | 37.98 | 2.37 | 2 |

### Single crystal X-ray diffraction

The crystal structure of co-crystal of agomelatine - maleic acid (1:1), form B, has been determined from single crystal X-ray diffraction data. The colourless crystal used (0.37 × 0.26 × 0.25 mm) was obtained from the vapour diffusion of a solution in methanol of agomelatine and maleic acid in a toluene atmosphere.

Analysis was performed at room temperature using a Bruker Smart Apex diffractometer with graphite monochromated Mo K_{α} radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0).

The structure was solved with direct methods and least-squares refinement of Fₒ² against all measured intensities was carried out (program used: SHELXTL-NT 6.1). All non-hydrogen atoms were refined with anisotropic displacement parameters.

Relevant structural data:

| Crystal system | Monoclinic |
|---|---|
| Space group | *P*2₁/*a* |
| a (Å) | 7.5055(6) |
| b (Å) | 20.5934(16) |
| c (Å) | 12.0880(9) |
| β (°) | 99.220(1) |
| Volume (Å³) | 1844.2(2) |
| Z | 4 |
| D calc. (Mg/m³) | 1.294 |
| N. of refl. | 4481 |
| Refl. with I > 2σ(I) | 2553 |
| R (I > 2σ(I)) | 0.0701 |

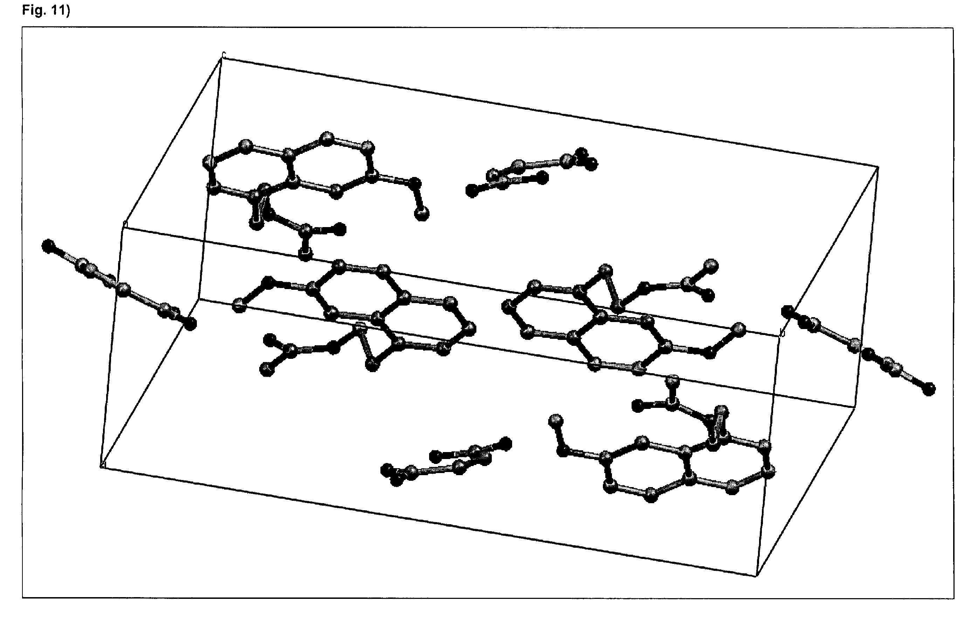

The unit cell contents of this crystal structure are depicted in figure 11 (hydrogen atoms have been omitted for clarity; program used: *Mercury* 2.3, C.F. Macrae, I.J. Bruno, J.A. Chisholm, P.R. Edgington, P. McCabe, E. Pidcock, L. Rodriguez-Monge, R. Taylor, J. van de Streek and P.A. Wood, J. Appl. Cryst., 41, 2008, 466-470).

Positions of peaks in XRPD diffractogram simulated from single crystal data are almost identical to those in the experimental one presented above.

### Example 4: Agomelatine - Gentisic acid (1:1) co-crystal

### Slurrying in toluene

To a round-bottom flask equipped with magnetic stirrer containing agomelatine (1.3 g, 5.34 mmol) and gentisic acid (906 mg, 5.88 mmol, 1.1 eq), was added toluene (4.4 mL). The resultant suspension was stirred at room temperature for 17 hours. The solid was filtered with a sintered funnel (porosity 4), washed with tert-butyl methyl ether (2 × 2.2 mL) and diethyl ether (1 × 4 mL and 1 × 3 mL) and dried under vacuum at room temperature to give co-crystal of agomelatine - gentisic acid (1:1) as a white solid (1.38 g, 65 % yield).

In a lower scale the same experiment was carried out with diethyl ether and isobutyl acetate.

### Wet grinding with dichloromethane (solvent assisted grinding)

To a 2 mL eppendorf containing agomelatine (31 mg, 0.127 mmol), gentisic acid (19 mg, 0.125 mmol) and three 4 mm stainless steel grinding balls, two drops of dichloromethane were added. The reactor was stirred 45 minutes at a rate of 30 Hz (3 × 15 minutes) giving co-crystal of agomelatine - gentisic acid (1:1) as a white solid (50 mg, quantitative yield).

### ¹H NMR

Proton nuclear magnetic resonance analyses were recorded in deuterated methanol (MeOD) in a Varian Mercury 400 spectrometer, equipped with a broadband probe ATB 1H/19F/X of 5 mm. Spectra were acquired dissolving 5-10 mg of sample in 0.6 mL of deuterated solvent.

¹H NMR (MeOD, 400 MHz): δ = 7.74 (d, *J* = 9.0 Hz, 1H); 7.65 (d, *J* = 7.8 Hz, 1H); 7.52 (d, *J* = 2.3 Hz, 1H); 7.30 (d, *J* = 7.0 Hz, 1H); 7.26-7.20 (m, 2H); 7.12 (dd, *J* = 9.0 Hz and 2.3 Hz, 1H); 6.95 (dd, *J* = 9.0 Hz and 3.1 Hz, 1H); 6.76 (d, *J* = 9.0 Hz, 1H); 3.96 (s, 3H); 3.53-3.45 (m, 2H); 3.25-3.18 (m, 2H); 1.93 (s, 3H).

### IR

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3390.0 (s,br), 3010.6 (m), 2362.0 (m,br), 1926.7 (s,br), 1660.2 (s), 1557.8 (m), 1510.0 (m), 1438.1 (m), 1377.2 (m), 1345.3 (m), 1287.7 (m), 1218.1 (m), 940.8 (s), 901.4 (s), 856.8 (s), 837.6 (s), 798.2 (s), 759.9 (m), 676.8 (s), 549.3 (m), 454.9 (s) cm⁻¹.

### DSC

DSC analyses were recorded with a Mettler DSC822^{e}. A sample of 3.3480 mg was weighed into 40 µL aluminium crucible with a pinhole lid and was heated, under nitrogen (50 mL/min), at 10 °C/min from 30 to 300 °C.

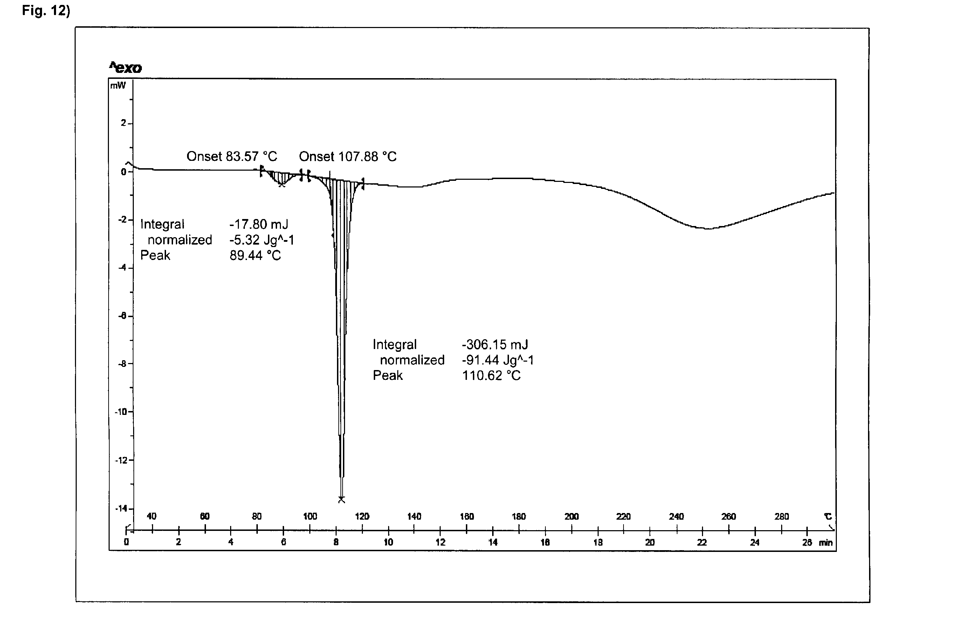

The novel type of crystal of the present invention is characterized in that the endothermic sharp peak corresponding to the melting point has an onset at 107.88 °C (fusion enthalpy - 91.44 J/g), measured by DSC analysis (10 °C/min) (see figure 12). (Another peak with an onset at 83.57°C could be due to the presence of a small crystalline impurity)

### TG

Thermogravimetric analyses were recorded in a thermogravimetric analyzer Mettler TGA/SDTA851^{e}. A sample of 14.1796 mg was weighed into a 70 µL alumina crucible with a pinhole lid and was heated at 10 °C/min from 30 to 200 °C, under nitrogen (50 mL/min).

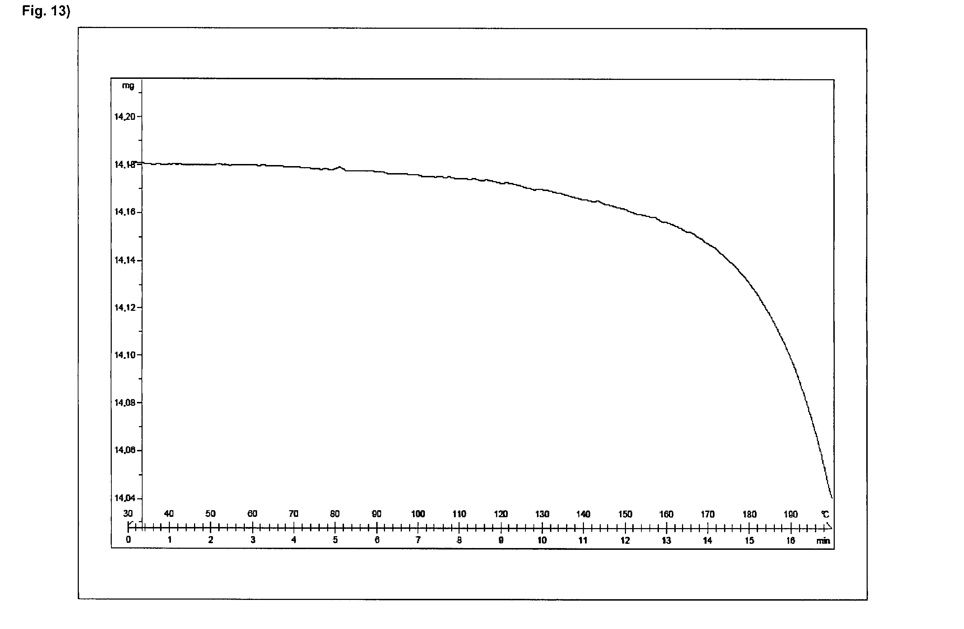

The TG analysis of the co-crystal of agomelatine - gentisic acid (1:1) according to the invention shows no weight loss at temperatures lower than the melting point (see figure 13).

### XRPD

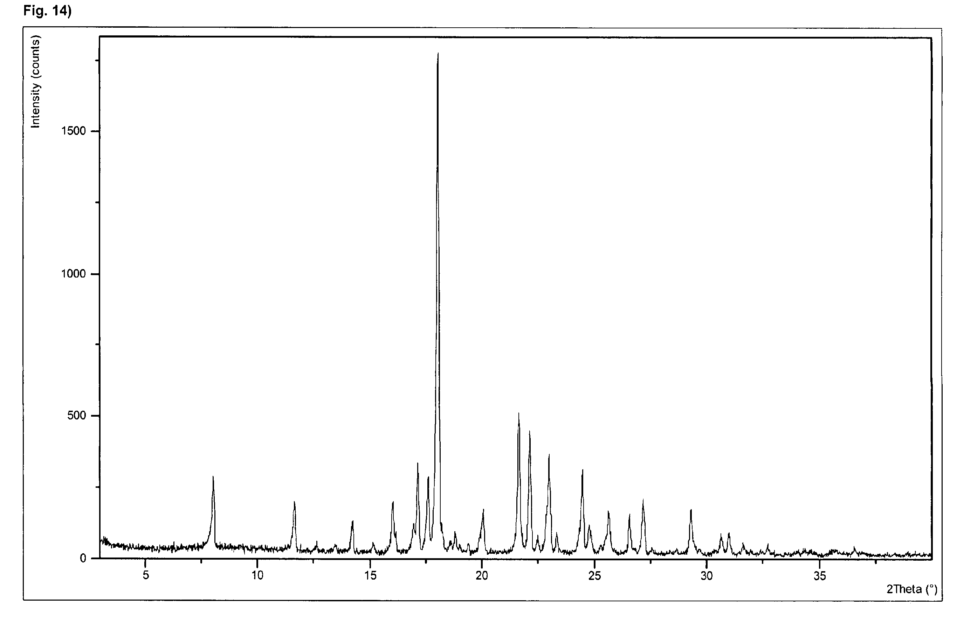

XRPD analysis was performed using a Philips X'Pert diffractometer with Cu K_{α} radiation in Bragg-Brentano geometry. The system is equipped with a monodimensional, real time multiple strip detector. The diffractogram was recorded from 3° to 40° (2θ) at a scan rate of 17.6° per minute (see figure 14).

List of selected peaks (only peaks with relative intensity greater than or equal to 1 % are indicated):

| **2**θ **(°)** | **d (**Å**)** | **l (%)** | | **2**θ **(°)** | **d (**Å**)** | **l (%)** |
|---|---|---|---|---|---|---|
| 8.06 | 10.97 | 11 | | 22.46 | 3.96 | 2 |
| 11.66 | 7.59 | 10 | | 22.98 | 3.87 | 20 |
| 12.60 | 7.03 | 1 | | 23.33 | 3.81 | 4 |
| 14.24 | 6.22 | 6 | | 24.46 | 3.64 | 16 |
| 15.16 | 5.84 | 2 | | 24.77 | 3.59 | 5 |
| 16.03 | 5.53 | 10 | | 25.64 | 3.47 | 8 |
| 16.94 | 5.23 | 5 | | 26.56 | 3.36 | 8 |
| 17.15 | 5.17 | 16 | | 27.17 | 3.28 | 11 |
| 17.60 | 5.04 | 15 | | 29.27 | 3.05 | 9 |
| 18.05 | 4.91 | 100 | | 30.64 | 2.92 | 3 |
| 18.81 | 4.72 | 3 | | 30.96 | 2.89 | 4 |
| 19.39 | 4.58 | 2 | | 31.61 | 2.83 | 2 |
| 20.04 | 4.43 | 8 | | 32.71 | 2.74 | 1 |
| 21.63 | 4.11 | 28 | | 36.55 | 2.46 | 1 |
| 22.13 | 4.02 | 24 | | | | |

### Single crystal X-ray diffraction

The crystal structure of co-crystal of agomelatine - gentisic acid (1:1) has been determined from single crystal X-ray diffraction data. The colourless prism used (0.26 × 0.12 × 0.09 mm) was obtained from the evaporation of a solution of agomelatine and gentisic acid in methanol.

Analysis was performed at room temperature using a Bruker Smart Apex diffractometer with graphite monochromated Mo K_{α} radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0).

The structure was solved with direct methods and least-squares refinement of Fₒ² against all measured intensities was carried out (program used: SHELXTL-NT 6.1). All non-hydrogen atoms were refined with anisotropic displacement parameters.

Relevant structural data:

| Crystal system | Monoclinic |
|---|---|
| Space group | P2₁/c |
| a (Å) | 11.3499(13) |
| b (Å) | 11.0448(14) |
| c (Å) | 16.803(2) |
| β (°) | 105_{.}000(3) |
| Volume (Å³) | 2034.6(4) |
| Z | 4 |
| D calc. (Mg/m³) | 1.297 |
| N. of refl. | 4919 |
| Refl. with > 2σ(I) | 1993 |
| R(I > 2σ(I)) | 0.0648 |

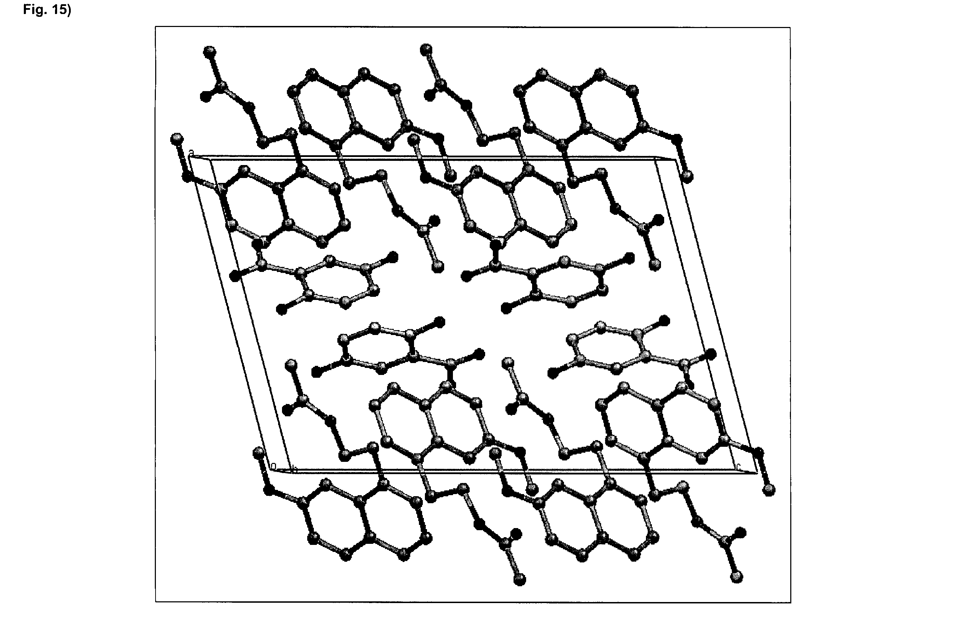

The unit cell contents of this crystal structure are depicted in figure 15 (hydrogen atoms have been omitted for clarity; program used: *Mercury* 2.3, C.F. Macrae, I.J. Bruno, J.A. Chisholm, P.R. Edgington, P. McCabe, E. Pidcock, L. Rodriguez-Monge, R. Taylor, J. van de Streek and P.A. Wood, J. Appl. Cryst., 41, 2008, 466-470).

Positions of peaks in XRPD diffractogram simulated from single crystal data are almost identical to those in the experimental one presented above.

### Example 5: Agomelatine - Citric acid (1:1) co-crystal

### Precipitation in acetone/heptane

To a round-bottom flask equipped with magnetic stirrer containing agomelatine (1 g, 4.11 mmol) and citric acid (790 mg, 4.11 mmol, 1.0 eq), was added acetone (5 mL). To the resultant solution was added heptane (16.5 mL) and a white precipitate was formed in few minutes. The mixture was stirred at room temperature for 16 hours. The solid was filtered with a sintered funnel (porosity 4), washed with a cold mixture of acetone:heptane (1:3, 40 mL) and dried under vacuum at room temperature to give co-co-crystal of agomelatine - citric acid (1:1) as a white solid (1.59 g, 89 % yield).

### Slow evaporation from acetone

To a vial containing agomelatine (30 mg, 0.12 mmol) and citric acid (24 mg, 0.12 mmol, 1.0 eq), was added acetone (0.5 mL). The resultant solution was slowly evaporated at room temperature. After complete evaporation, co-crystal of agomelatine - citric acid (1:1) was obtained as a white solid (54 mg, quantitative yield).

The same experiment was carried out with methanol and dioxane.

### ¹H NMR

Proton nuclear magnetic resonance analyses were recorded in deuterated methanol (MeOD) in a Varian Mercury 400 spectrometer, equipped with a broadband probe ATB 1H/19F/X of 5 mm. Spectra were acquired dissolving 5-10 mg of sample in 0.6 mL of deuterated solvent.

¹H NMR (MeOD, 400 MHz): δ = 7.75 (d, *J* = 9.0 Hz, 1H); 7.65 (d, *J* = 7.8 Hz, 1H); 7.53 (d, *J* = 2.3 Hz, 1H); 7.31 (d, *J* = 7.0 Hz, 1H); 7.24 (dd, *J* = 7.8 Hz and 7.0 Hz, 1H); 7.12 (dd, *J* = 9.0 Hz and 2.3 Hz, 1H); 3.96 (s, 3H); 3.49 (dd, *J* = 8.4 Hz and 7.0 Hz, 2H); 3.22 (dd, *J* = 7.0 Hz and 8.4 Hz, 2H); 2.91 (d, *J* = 15.6 Hz, 2H); 2.79 (d, *J* = 15.6 Hz, 2H); 1.93 (s, 3H).

### IR

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3498.3 (s), 3319.3 (s), 3202.4 (m,br), 1760.8 (s), 1702.0 (m), 1624.8 (m), 1584.0 (m), 1509.7 (s), 1447.6 (m), 1383.0 (m), 834.2 (m), 815.1 (m), 758.5 (m), 633.9 (m), 508.2 (m)cm⁻¹.

### DSC

DSC analyses were recorded with a Mettler DSC822^{e}. A sample of 0.9230 mg was weighed into 40 µL aluminium crucible with a pinhole lid and was heated, under nitrogen (50 mL/min), at 10 °C/min from 30 to 190 °C.

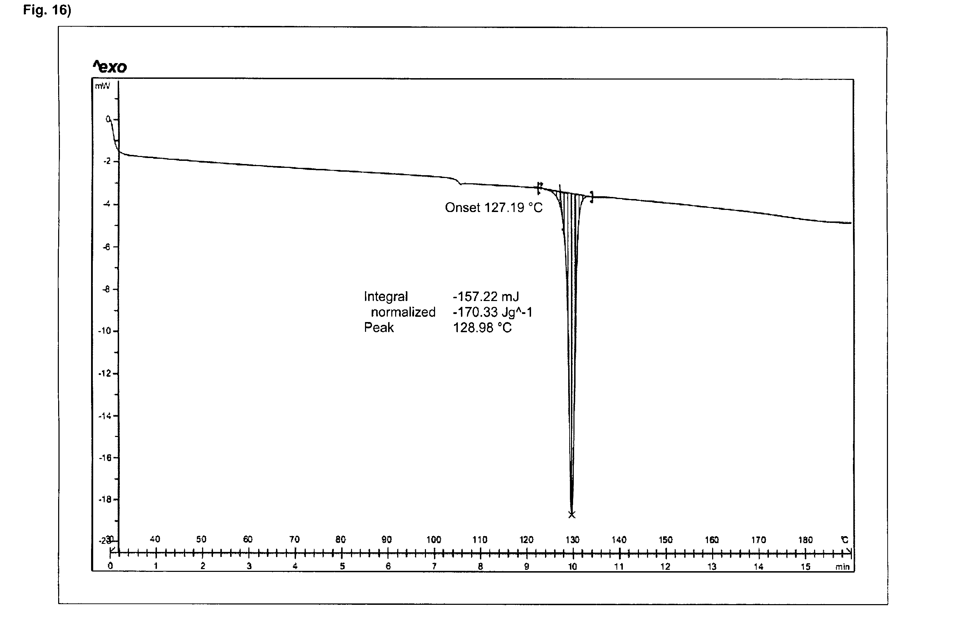

The novel type of crystal of the present invention is characterized in that the endothermic sharp peak corresponding to the melting point has an onset at 127.19 °C (fusion enthalpy - 170.33 J/g), measured by DSC analysis (10 °C/min) (see figure 16).

### TG

Thermogravimetric analyses were recorded in a thermogravimetric analyzer Mettler TGA/SDTA851^{e}. A sample of 4.0096 mg was weighed into a 70 µL alumina crucible with a pinhole lid and was heated at 10 °C/min from 30 to 200 °C, under nitrogen (50 mL/min).

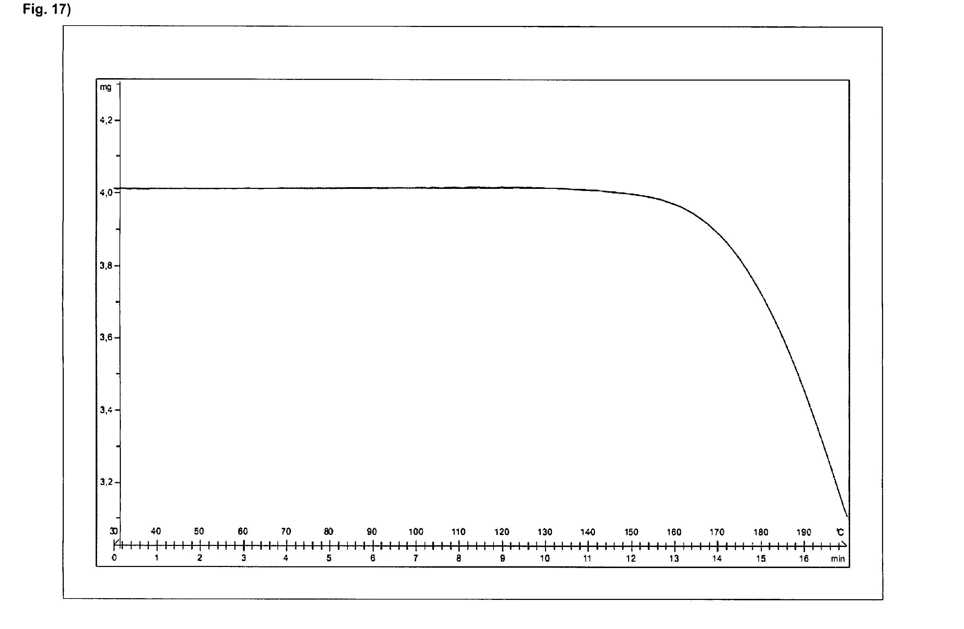

The TG analysis of the co-crystal of agomelatine - citric acid (1:1) according to the invention shows no weight loss at temperatures lower than the melting point (see figure 17).

### XRPD

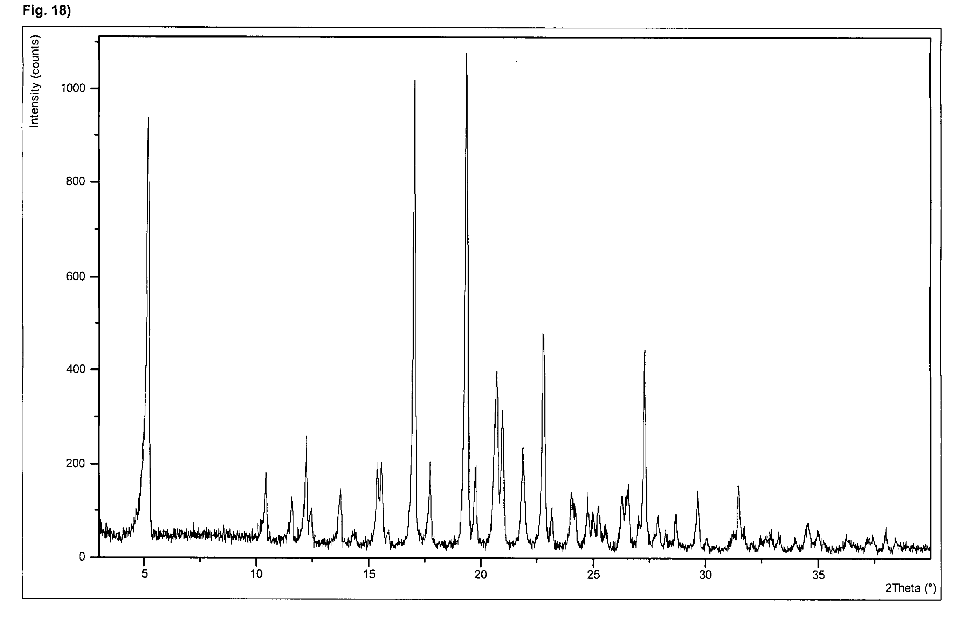

XRPD analysis was performed using a Philips X'Pert diffractometer with Cu K_{α} radiation in Bragg-Brentano geometry. The system is equipped with a monodimensional, real time multiple strip detector. The diffractogram was recorded from 3° to 40° (2θ) at a scan rate of 17.6° per minute (see figure 18).

List of selected peaks (only peaks with relative intensity greater than or equal to 1 % are indicated):

| **2**θ **(°)** | **d (**Å**)** | **l (%)** | | **2**θ **(°)** | **d (**Å**)** | **l (%)** |
|---|---|---|---|---|---|---|
| 5.22 | 16.90 | 79 | | 24.99 | 3.56 | 6 |
| 10.42 | 8.49 | 11 | | 25.23 | 3.53 | 8 |
| 11.58 | 7.64 | 8 | | 25.54 | 3.49 | 4 |
| 12.24 | 7.23 | 16 | | 26.26 | 3.39 | 9 |
| 12.43 | 7.12 | 7 | | 26.57 | 3.35 | 10 |
| 13.74 | 6.44 | 11 | | 27.28 | 3.27 | 37 |
| 14.35 | 6.17 | 2 | | 27.88 | 3.20 | 5 |
| 15.38 | 5.76 | 15 | | 28.23 | 3.16 | 2 |
| 15.55 | 5.70 | 16 | | 28.66 | 3.11 | 5 |
| 17.06 | 5.20 | 94 | | 29.66 | 3.01 | 10 |
| 17.74 | 5.00 | 16 | | 31.42 | 2.85 | 11 |
| 19.36 | 4.58 | 100 | | 32.72 | 2.74 | 2 |
| 19.77 | 4.49 | 16 | | 33.25 | 2.69 | 2 |
| 20.72 | 4.29 | 35 | | 33.97 | 2.64 | 2 |
| 20.98 | 4.23 | 24 | | 34.54 | 2.60 | 5 |
| 21.87 | 4.06 | 20 | | 35.01 | 2.56 | 3 |
| 22.80 | 3.90 | 43 | | 36.28 | 2.48 | 2 |
| 23.15 | 3.84 | 7 | | 37.33 | 2.41 | 1 |
| 24.05 | 3.70 | 11 | | 37.98 | 2.37 | 3 |
| 24.75 | 3.60 | 8 | | 38.44 | 2.34 | 2 |

### Single crystal X-ray diffraction

The crystal structure of co-crystal of agomelatine - citric acid (1:1) has been determined from single crystal X-ray diffraction data. The colourless plate used (0.28 × 0.17 × 0.17 mm) was obtained from the vapour diffusion of a solution in heptane of agomelatine and citric acid in a heptane atmosphere.

Analysis was performed at room temperature using a Bruker Smart Apex diffractometer with graphite monochromated Mo K_{α} radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0).

The structure was solved with direct methods and least-squares refinement of Fₒ² against all measured intensities was carried out (program used: SHELXTL-NT 6.1). All non-hydrogen atoms were refined with anisotropic displacement parameters.

Relevant structural data:

| Crystal system | Monoclinic |
|---|---|
| Space group | P2₁/c |
| a (Å) | 7.8396(6) |
| b (Å) | 33.892(2) |
| c (Å) | 8.2910(6) |
| β (°) | 109.376(1) |
| Volume (Å³) | 2078.1(3) |
| Z | 4 |
| D calc. (Mg/m³) | 1.392 |
| N. of refl. | 5085 |
| Refl. with I > 2σ(I) | 3486 |
| R(I > 2σ(I)) | 0.0496 |

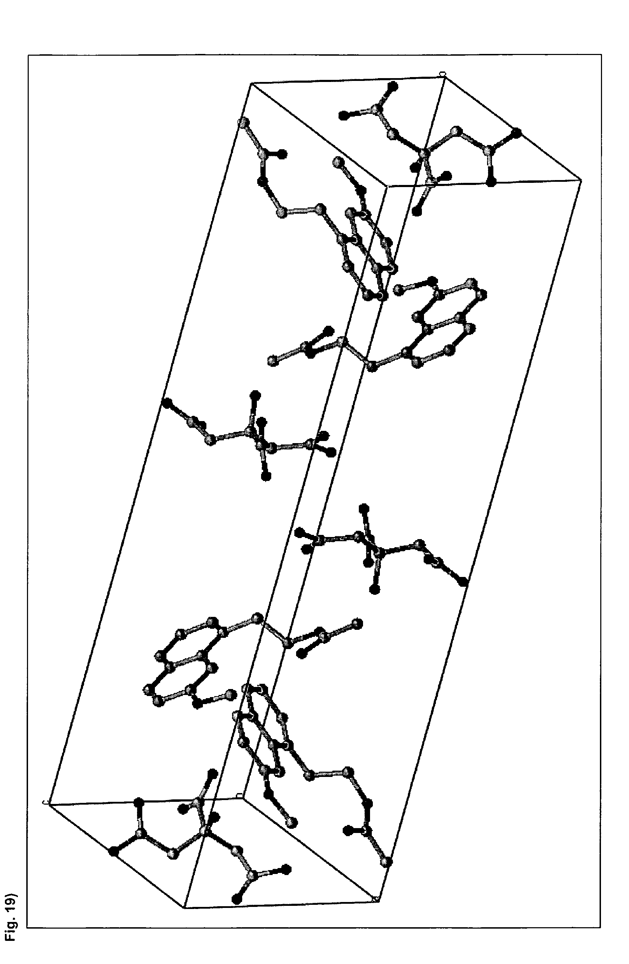

The unit cell contents of the crystal structure of form 189 are depicted in figure 19 (hydrogen atoms have been omitted for clarity; program used: *Mercury* 2.3, C.F. Macrae, I.J. Bruno, J.A. Chisholm, P.R. Edgington, P. McCabe, E. Pidcock, L. Rodriguez-Monge, R. Taylor, J. van de Streek and P.A. Wood, J. Appl. Cryst., 41, 2008, 466-470).

Positions of peaks in XRPD diffractogram simulated from single crystal data are almost identical to those in the experimental one presented above.

### Example 6: Determination of Intrinsic Dissolution Rate (IDR) test of the co-crystals from examples 4 and 5.

The aim of this study was to measure intrinsic dissolution rate (IDR) of some crystalline forms of the present invention in order to compare them with the IDR of the agomelatine. Intrinsic dissolution rate is defined as the dissolution rate of pure substance under the condition of constant surface area.

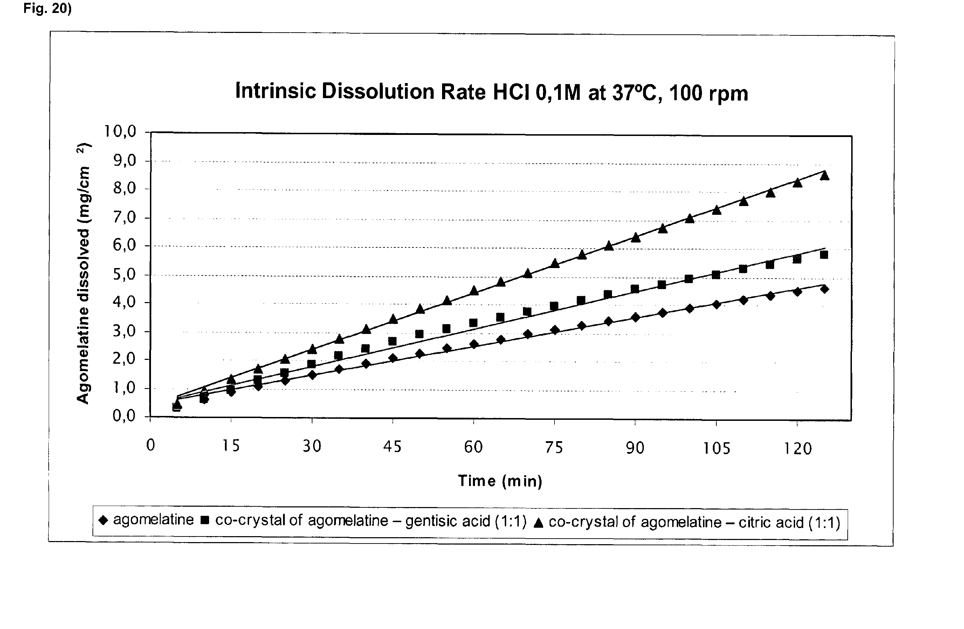

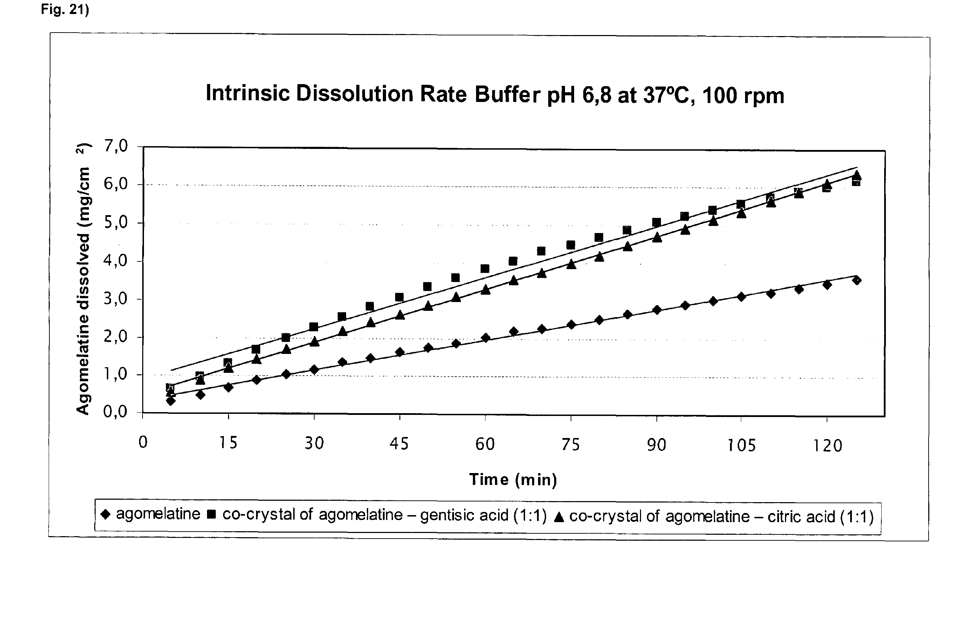

Figures 20 and 21 show the results of intrinsic dissolution rate performed with co-crystals obtained in examples 4 and 5. The IDR behaviour of agomelatine and the co-crystal of agomelatine - gentisic acid (1:1), agomelatine - citric acid (1:1) from the present invention was tested in USP apparatus 2 (paddles) at 37°C and 100 rpm with 2 hours in a solution of 0,1 N HCl (figure 20) and 2 hours in a buffer solution at pH 6.8 (figure 21). The intrinsic dissolution rate was calculated from the slope of the straight line obtained.

A summary of values of IDR obtained is shown in table 1.

**Table 4. Summary of IDR results**

| **Substances** | **Media** | **IDR (mg/cm²·s)** | **Ratio** |
|---|---|---|---|
| agomelatine | HCl 0.1M | 0.0349 | - |
| agomelatine-citric acid (1:1) co-crystal | | 0.0670 | 1.92 |
| agomelatine - gentisic acid (1:1) co-crystal | | 0.0447 | 1.28 |
| agomelatine | Buffer pH 6.8 | 0.0267 | - |
| agomelatine-citric acid(1:1) co-crystal | | 0.0470 | 1.76 |
| agomelatine-gentisic acid (1:1) co-crystal | | 0.0453 | 1.70 |

Both tests prove IDR of agomelatine contained in the co-crystals increased, in both media, to almost twice compare to the IDR of agomelatine API.

## Claims

1. A co-crystal comprising agomelatine either as a free base or as a physiologically salt and at least one co-crystal-former either as a free base or as a physiologically acceptable salt with the proviso that
• a co-crystal of agomelatine with acetic acid, and
• a co-crystal of agomelatine with ethylene glycol
are excluded.

2. The co-crystal according to claim 1, wherein the co-crystal-former is selected from either carbamides or carbonic acids, preferably is selected from either carbamides or carbonic acids with less than 9 carbon atoms, most preferably is selected from the group consisting of urea, maleic acid, gentisic acid, citric acid or their stereoisomers.

3. The co-crystal according to any of claims 1 or 2, wherein the co-crystal is selected from agomelatine - urea (1:1), agomelatine - maleic acid (1:1) form A, agomelatine - maleic acid (1:1) form B, agomelatine - gentisic acid (1:1); or agomelatine - citric acid (1:1).

4. The co-crystal according to any of the preceding claims, wherein the ratio between agomelatine and co-crystal former is chosen in such a way that if compared to either agomelatine alone and/or to a mixture of agomelatine and co-crystal former
• the solubility of the co-crystal is increased; and/or
• the dose response of the co-crystal is increased; and/or
• the efficacy of the co-crystal is increased; and/or
• the dissolution of the co-crystal is increased; and/or
• the bioavailability of the co-crystal is increased; and/or
• the stability of the co-crystal is increased; and/or
• the hygroscopicity of the co-crystal is decreased; and/or
• the form diversity of the co-crystal is decreased; and/or
• the morphology of the co-crystal is modulated.

5. The co-crystal according to claim 1 or 2, wherein the molecular ratio between agomelatine and co-crystal-former is 1:1.

6. The co-crystal of agomelatine - urea (1:1) according to claim 3, **characterized in that** the endothermic sharp peak corresponding to the melting point has an onset at 135 °C, preferably at 134.6 °C, most preferably at 134.64 °C; or
**characterized in that** it shows an X-Ray powder diffraction pattern with peaks [2θ] at 15.59 and 22.81, preferably with peaks [2θ] at 15.59, 19.53, 22.81, 28.04, and 31.43, more preferably with peaks [2θ] at 9.54, 13.34, 15.59, 17.65, 19.53, 21.35, 22.81, 28.04, and 31.43, most preferably with peaks [2θ] at 9.54, 10.70, 11.32, 13.34, 15.59, 17.65, 18.93, 19.13, 19.53, 21.35, 22.04, 22.81, 24.32, 24.81, 25.30, 26.33, 28.04, 29.27, 31.43 and 33.23; or
**characterized in that** it has a monoclinic unit cell with the following dimensions:
a = 9.72 Å,
b = 9.24 Å, and
c = 18.53 Å,
with an angle β of 102.00 °.

7. The co-crystal of agomelatine - maleic acid (1:1) form A according to claim 3, **characterized in that** the endothermic sharp peak corresponding to the melting point has an onset at 68 °C, preferably at 67.8 °C, most preferably at 67.81 °C; or
**characterized in that** it shows an X-Ray powder diffraction pattern with peaks [2θ] at 15.93, 25.83, and 26.20, preferably with peaks [2θ] at 14.61, 15.93, 17.63, 23.43, 25.83, and 26.20, more preferably with peaks [2θ] at 8.78, 11.26, 14.61, 15.93, 17.63, 19.69, 23.43, 25.83, and 26.20, most preferably with peaks [2θ] at 8.78, 11.26, 11.70, 12.45, 14.61, 15.44, 15.93, 17.11, 17.63, 19.69, 20.23, 21.18, 22.23, 23.43, 24.40, 25.08, 25.83, 26.20, 27.03, 29.07, 29.50, 30.56, 32.07, 33.31 and 35.68.

8. The co-crystal of agomelatine - maleic acid (1:1) form B according to claim 3, **characterized in that** the endothermic sharp peak corresponding to the melting point has an onset at 73 °C, preferably at 73.2 °C, most preferably at 73.18 °C; or
**characterized in that** it shows an X-Ray powder diffraction pattern with peaks [2θ] at 11.31, 15.42, 17.13, 17.30, 18.02, and 24.32, preferably with peaks [2θ] at 8.57, 11.31, 15.42, 17.13, 17.30, 18.02, 18.73, 23.93, 24.32, 25.48, and 26.22, more preferably with peaks [2θ] at 8.57, 11.31, 15.42, 17.13, 17.30, 17.57, 18.02, 18.73, 21.80, 22.93, 23.93, 24.32, 25.48, 26.22, 27.24, and 30.25, most preferably with peaks [2θ] at 7.37, 8.57, 11.31, 13.64, 14.67, 15.42, 15.62, 17.13, 17.30, 17.57, 18.02, 18.73, 19.48, 21.80, 22.24, 22.93, 23.93, 24.32, 25.48, 26.22, 26.53, 26.97, 27.24, 27.57, 28.04, 29.31, 30.25, 31.51, 32.11, 34.27, 35.20, 36.51, 37.31 and 37.98; or
**characterized in that** it has a monoclinic unit cell with the following dimensions:
a = 7.51 Å,
b = 20.59 Å, and
c = 12.09 Å,
with an angle β of 99.22 °.

9. The co-crystal of agomelatine - gentisic acid (1:1) according to claim 3, **characterized in that** the endothermic sharp peak corresponding to the melting point has an onset at 108 °C, preferably at 107.9 °C, most preferably at 107.88 °C; or
**characterized in that** it shows a X-Ray powder diffraction pattern with peaks [2θ] at 18.05, 21.63, and 22.98, preferably with peaks [2θ] at 17.15, 18.05, 21.63, 22.13, 22.98, and 24.46, more preferably with peaks [2θ] at 8.06, 11.66, 16.03, 17.15, 17.60, 18.05, 21.63, 22.13, 22.98, 24.46, and 27.17, most preferably with peaks [2θ] at 8.06, 11.66, 12.60, 14.24, 15.16, 16.03, 16.94, 17.15, 17.60, 18.05, 18.81, 19.39, 20.04, 21.63, 22.13, 22.46, 22.98, 23.33, 24.46, 24.77, 25.64, 26.56, 27.17, 29.27, 30.64, 30.96, 31.61, 32.71 and 36.55; or
**characterized in that** it has a monoclinic unit cell with the following dimensions:
a = 11.35 Å,
b = 11.04 Å, and
c = 16.80 Å,
with an angle β of 105.00 °.

10. The co-crystal of agomelatine - citric acid (1:1) according to claim 3, **characterized in that** the endothermic sharp peak corresponding to the melting point has an onset at 127 °C, preferably at 127.2 °C, most preferably at 127.16 °C; or
**characterized in that** it shows a X-Ray powder diffraction pattern with peaks [2θ] at 5.22, 17.06, 19.36, 22.80, and 27.28, preferably with peaks [2θ] at 5.22, 17.06, 19.36, 20.72, 20.98, 21.87, 22.80, and 27.28, more preferably with peaks [2θ] at 5.22, 10.42, 12.24, 13.74, 15.38, 15.55, 17.06, 17.74, 19.36, 19.77, 20.72, 20.98, 21.87, 22.80, 24.05, 26.57, 27.28, 29.66, and 31.42, most preferably with peaks [2θ] at 5.22, 10.42, 11.58, 12.24, 12.43, 13.74, 14.35, 15.38, 15.55, 17.06, 17.74, 19.36, 19.77, 20.72, 20.98, 21.87, 22.80, 23.15, 24.05, 24.75, 24.99, 25.23, 25.54, 26.26, 26.57, 27.28, 27.88, 28.23, 28.66, 29.66, 31.42, 32.72, 33.25, 33.97, 34.54, 35.01, 36.28, 37.33, 37.98 and 38.44; or **characterized in that** it has a monoclinic unit cell with the following dimensions:
a = 7.84 Å,
b = 33.89 Å, and
c=8.29 Å,
with an angle β of 109.38 °.

11. Process for the production of a co-crystal according to any of claims 1 to 10 comprising the steps of:
(a) dissolving or suspending agomelatine and co-crystal former in a solvent or mixture of solvents,
(b) optionally mixing or stirring the solution or suspension,
(c) optionally before, during or after step (b) cooling or keeping the mixed solution/suspension to or at ambient temperature or below;
(d) optionally filtering-off and/or washing the resulting solid with a solvent, and
(e) drying the solid optionally at ambient temperature and/or vacuum.

12. Medicament comprising at least one co-crystal according to any of claims 1 to 10 and optionally one or more pharmaceutically acceptable excipients.

13. Pharmaceutical composition **characterized in that** it comprises a therapeutically effective amount of the co-crystal according to any of claims 1 to 10 in a physiologically acceptable medium.

14. The co-crystal according to any one of claims 1 to 10 for use in the treatment of depression, especially for the treatment of major depressive episodes.
